# EUROPÄISCHE PATENTANMELDUNG

(11) **EP 4 817 521 A1**
(43) Veröffentlichungstag der Anmeldung: **07.10.2026**
(21) Anmeldenummer: 25167322.4
(22) Anmeldetag: 31.03.2025
(51) Int. Cl.: A61B 5/256, A61F 5/56, A61B 5/296

(54) **GESUNDHEITSÜBERWACHUNGSSYSTEM MIT MUNDSTÜCK**

(71) Anmelder: Lorenz, Ernst O., 61381 Friedrichsdorf (DE)
(72) Erfinder: Lorenz, Ernst O., 61381 Friedrichsdorf (DE)
(74) Vertreter: Kilian Kilian & Partner mbB

(57) **Zusammenfassung**

Die Erfindung betrifft ein Gesundheitsüberwachungssystem zur Analyse und/oder Beeinflussung des Gesundheitszustands und/oder des Schlafverhaltens einer Person aufweisend ein Mundstück (1) und eine Auswertungseinrichtung (14, 54), zumindest einen oralen Sensor (16, 18, 20, 22, 24, 26, 28, 30, 32, 34) zur Messung eines oralen Parameters, wobei das Gesundheitsüberwachungssystem zumindest einen externen Sensor (56, 58, 60, 62, 64, 66, 68) aufweist, der kein Teil des Mundstücks (1) ist und der zur Messung zumindest eines externen Parameters vorgesehen ist, wobei die Auswertungseinrichtung (14, 54) derart programmiert ist, dass eine Analyse des Gesundheitszustands und/oder des Schlafverhaltens der Person unter Berücksichtigung sowohl der Auswertung der oralen Messdaten als auch der Auswertung der externen Messdaten erfolgt.

Die Erfindung betrifft auch Verfahren zur Analyse und/oder Beeinflussung des Gesundheitszustands und/oder des Schlafverhaltens einer Person.

## Beschreibung

Die Erfindung betrifft ein Gesundheitsüberwachungssystem sowie ein Verfahren zur Messung und gegebenenfalls auch zur Beeinflussung des Gesundheitszustands und/oder des Schlafverhaltens einer Person. Das Gesundheitsüberwachungssystem ist insbesondere und erfindungsgemäß bevorzugt zur Analyse des Gesundheitszustands und/oder des Schlafverhaltens und zur Messung von Parametern hierzu, die den Gesundheitszustand und/oder das Schlafverhalten beeinflussen, vorgesehen, und kann folglich auch ohne weiteres während des Wachens eingesetzt werden, sofern das Mundstück getragen wird. Daher kann eine Überwachung von gesundheitsrelevanten Parametern mit dem erfindungsgemäßen Gesundheitsüberwachungssystem und dem erfindungsgemäßen Verfahren auch während des Wachens erfolgen. Grundsätzlich lassen sich das Gesundheitsüberwachungssystem mit dem Mundstück und das Verfahren auch auf Tiere anwenden oder anpassen.

Aufbissschienen werden zur Behandlung von Bruxismus (dem Knirschen mit den Zähnen während des Schlafens) verwendet. Dazu wird üblicherweise ein Abdruck des Mundraums von einem Zahnarzt genommen und eine umlaufende Schiene entlang des Zahnbogens einer Person erzeugt, die auf die okklusalen Seiten der Zähne der Person aufgesteckt werden kann. Diese Aufbissschiene wird dann als Mundstück in den Mundraum der Person eingesetzt und während des Schlafens getragen, um die Auswirkungen des Bruxismus zu verringern und insbesondere einer (weiteren) Abnutzung des Zahnschmelzes vorzubeugen. Aufbissschienen werden dabei üblicherweise aus Kunststoff gefertigt.

Zahnspangen werden von Zahnärzten eingesetzt, um Fehlstellungen der Zähne einer Person zu korrigieren. Zur Herstellung von temporären Zahnspangen, die nicht fest mit den Zähnen verbunden werden, werden ebenfalls Abdrücke des Mundraums aufgenommen und basierend auf den Abdrücken die temporären Zahnspangen konstruiert.

Neben einer reinen Verwendung als Aufbissschiene sind in letzter Zeit zusätzliche Anwendungen für Aufbissschienen in Betracht gezogen worden, die im Zusammenhang mit oral einsetzbaren Mundstücken vorgeschlagen wurden. Die DE 10 2006 007 335 B3 offenbart ein Mundstück in Form einer Schiene entlang eines Zahnbogens, die eine Vielzahl von Sensoren sowie eine Energieversorgung und einen Vibrationsalarm enthält, um Bruxismus (das Knirschen mit den Zähnen während des Schlafens) zu therapieren. Ein weiteres System mit Mundstück zur Behandlung von Bruxismus ist aus der WO 2012/175634 A1 bekannt. Eine zweiteilige Zahnschiene zum Vorschieben des Unterkiefers, die mit Sensoren zur Überwachung von Schlafparametern bestückt ist, ist aus der US 2016/0324681 A1 bekannt. Dabei wird das Schnarchen einer Person akustisch gemessen und aufgenommen. Nachteilig ist an solchen Systemen, dass nur unmittelbar auf das Schnarchen reagiert werden kann und eine Aktivierung des Systems erfolgt, ohne dass andere Parameter oder personenspezifische Eigenschaften berücksichtigt werden. Dies führt mit hoher Wahrscheinlichkeit zu einer zu häufigen Aktivierung des Systems und damit zu einer zu häufigen Einflussnahme auf das Schlafverhalten der Person und damit zu einer unnötigen Beeinträchtigung des Schlafs der Person. Gleichzeitig wäre es wünschenswert, das Schlafverhalten so schonend wie möglich zu beeinflussen. Die US 2017/0128721 A1 offenbart in den Mundraum einsetzbare Schläuche mit Stimulations-Elektroden zur Applikation eines Medikaments und zum Messen eines Schluckvorgangs. Da mit diesem System lediglich ein Schluckvorgang angeregt werden soll, ist es zur Überwachung des Schlafverhaltens weniger gut geeignet.

Die US 2019/0091061 A1 und die US 2014/0135868 A1 offenbaren jeweils ein Mundstück mit mehreren oralen Sensoren und Stimulatoren, mit denen durch eine elektrisch über die Stimulatoren induzierte Versteifung der Zunge ein Schnarchen unterbunden werden soll. Auch bei diesem System ist es nachteilig, dass es zu einer unnötigen Aktivierung der Stimulatoren kommen kann und dadurch der Schlaf der Person unnötig beeinträchtigt wird. Zudem ist die Zahl von auf den Schlaf der Person einflussnehmenden Parametern, die mit dem System erfasst werden können, beschränkt und damit auch die Auswertung und das Erkennen von Schlafzuständen und Schlafverhalten der Person unvollständig.

Aus der US 2012/0240941 A1 ist ein System bekannt, mit dem Schlafstörungen wie Apnoe behandelt werden sollen. Dabei wird der Anwender mit einer Vibrationseinrichtung gewarnt, falls er eine ungünstige Schlafposition einnimmt. Die US 2015/0290465 A1 offenbart eine Vorrichtung zum drahtlosen Wiederaufladen eines Akkumulators eines implantierten Implantats. Das Implantat hat Aktoren zur neuronalen Stimulation zum Verhindern von Apnoe und Schnarchen. Andere Ursachen von Schlafapnoe und Schnarchen werden bei diesen Systemen nicht berücksichtigt. Eine möglichst vollständige Analyse des Schlafverhaltens ist dort nicht beabsichtigt.

Die WO 2021/097377 A1 offenbart eine mandibuläre Repositionierungsvorrichtung, die Stimulationselektroden aufweist. Mit der Vorrichtung wird eine Vielzahl von möglichen oralen Parametern vorgeschlagen, anhand derer der Schlaf der Person überwachbar sein soll. Das System soll vor allem über die Stellung des Unterkiefers eine Schlafapnoe und eine Sauerstoffuntersättigung im Blut der Person verhindern. Ein ähnliches System ist aus der WO 2023/240063 A2 bekannt. Nachteilig ist an beiden Dokumenten, dass Ursachen, die auf das Schlafverhalten Einfluss nehmen, nicht berücksichtigt werden und damit die Analyse des Schlafverhaltens trotz der Aufnahme vieler Parameter unvollständig bleibt. Insbesondere werden auf das Schlafverhalten der Person einflussnehmende Parameter nicht gemessen und so ein unvollständiges Bild gebildet. Die Ausrichtung des Unterkiefers wird daher unter Umständen ohne Notwendigkeit eingestellt und dadurch der Schlaf der Person unnötig beeinträchtigt.

Aus der EP 3 151 792 B1 ist ein System bekannt, mit dem Schlafstörungen wie Apnoe behandelt werden sollen. Das System umfasst jeweils eine Aufbissplatte für den Oberkiefer und für den Unterkiefer, die motorgetrieben gegeneinander bewegt werden können, um den Unterkiefer in eine Stellung zu bringen, der ein barrierefreies Atmen während des Schlafs ermöglicht. Zur Steuerung eines Stellglieds weist das System zusätzlich Sensoren auf, die die Körperfunktionen des Trägers überwachen. Dieses System hat den Nachteil, dass es zu einer zu häufigen Ansteuerung des Motors kommen kann und dadurch den Schlaf des Anwenders stören kann. Andererseits können zumindest einige sich anbahnende Atmungsstörungen nicht erkannt werden, so dass eine Beeinflussung zu spät erfolgt, wenn ihnen schonender begegnet werden könnte. Es ist immer wünschenswert den Schlaf des Anwenders möglichst immer nur dann zu beeinflussen und damit zu stören, falls dies wirklich notwendig ist.

Nachteilig an den bekannten Schlafüberwachungssystemen zur Messung oder zur Messung und Beeinflussung des Schlafverhaltens einer Person ist, dass meist eine zu spezielle Messung beziehungsweise eine unvollständige oder zu ungenaue Analyse des Schlafverhaltens erfolgt, bei der Einflussgrößen, die auf den Schlaf der Person Einfluss haben oder die Hinweise auf den Gesundheitszustand der Person zulassen, nicht vollständig oder nicht vollständig genug ermittelt werden. Zudem wird bei Systemen mit Aktoren, auf Basis der unvollständigen oder verbesserungsfähigen Datenlage eine Beeinflussung des Schlafverhaltens zu häufig und mit zu starken Mitteln erfolgen. Des Weiteren können andere behandlungsbedürftige Beeinträchtigungen oder Erkrankungen unerkannt bleiben.

Es ist immer wünschenswert, eine Störung des Schlafverhaltens der Person nur dann vorzunehmen, wenn dies wirklich notwendig ist und dabei möglichst die Anzahl der Eingriffe in das Schlafverhalten zu reduzieren. Des Weiteren wäre es wünschenswert, dass wenn Eingriffe unvermeidlich sind, diese so schonend wie möglich durchzuführen und auf diese Weise den Schlaf der Person so wenig wie möglich zu stören.

Der vorliegenden Erfindung liegt die Aufgabe zugrunde, wenigstens einen der vorgenannten Nachteile aus dem Stand der Technik oder einen sonstigen sich aus dem Stand der Technik ergebenden Nachteil zumindest teilweise zu überwinden. Insbesondere soll ein Gesundheitsüberwachungssystem und ein Verfahren gefunden werden, mit denen eine möglichst vollständige Analyse des Gesundheitszustands und/oder des Schlafverhaltens einer Person erfolgen kann. Bevorzugt soll gleichzeitig eine Beeinflussung des Schlafverhaltens auf Basis der Analysen möglich sein, wobei der Schlaf der Person dabei so wenig und so behutsam wie möglich und nötig beeinträchtigt werden soll. Hierzu soll ein möglichst ganzheitliches und detailliertes Bild des Gesundheitszustands und/oder des Schlafverhaltens der Person gewonnen werden.

Die Aufgaben der Erfindung werden zumindest teilweise gelöst durch ein Gesundheitsüberwachungssystem nach Anspruch 1 und durch ein Verfahren nach Anspruch 12. Bevorzugte Varianten werden mit den Unteransprüchen 2 bis 11 und 13 bis 15 beansprucht.

Zumindest eine der vorgenannten Aufgaben oder eine sich aus dem Stand der Technik ergebende Aufgabe wird also zumindest teilweise gelöst durch ein Gesundheitsüberwachungssystem zur Analyse eines Gesundheitszustands einer Person, insbesondere zur Analyse eines Schlafverhaltens einer Person, oder zur Analyse und Beeinflussung eines Gesundheitszustands einer Person, insbesondere des Schlafverhaltens einer Person, das Gesundheitsüberwachungssystem aufweisend ein Mundstück zum Einsetzen in den Mundraum der Person und eine Auswertungseinrichtung, wobei das Mundstück zumindest einen oralen Sensor aufweist, wobei der zumindest eine orale Sensor zur Messung zumindest eines oralen Parameters im Mundraum der Person vorgesehen ist, wobei die Auswertungseinrichtung zur Auswertung der oralen Messdaten des zumindest einen oralen Sensors vorgesehen ist, wobei das Gesundheitsüberwachungssystem zumindest einen externen Sensor aufweist, der kein Teil des Mundstücks ist, wobei der zumindest eine externe Sensor zur Messung zumindest eines externen Parameters in der unmittelbaren Umgebung der Person vorgesehen ist, wobei die Auswertungseinrichtung zur Auswertung der externen Messdaten des zumindest einen externen Sensors vorgesehen ist, wobei die Auswertungseinrichtung derart programmiert ist, dass eine Analyse des Gesundheitszustands und/oder des Schlafverhaltens der Person unter Berücksichtigung sowohl der Auswertung der oralen Messdaten als auch der Auswertung der externen Messdaten erfolgt.

Unter einem oralen Sensor wird vorliegend ein Sensor verstanden, der an dem Mundstück angeordnet ist. Bevorzugt befinden sich alle oralen Sensoren im Mundraum der Person, wenn das Mundstück ordnungsgemäß (wie vorgesehen) in den Mundraum der Person eigesetzt ist.

Bevorzugt weist das Mundstück mehrere orale Sensoren zur Messung unterschiedlicher oralen Parameter auf.

Unter einem externen Sensor sind solche Sensoren zu verstehen, die nicht am Mundstück angeordnet sind und die dazu geeignet sind, physikalische Parameter und Messwerte in der Umgebung der Person aufzunehmen. Die externen Sensoren sind folglich nicht zur Bestimmung von Körperfunktionen oder anderen sich auf die Person selbst beziehenden Größen vorgesehen. Die externen Sensoren sind zur Messung von physikalischen Größen und Werten geeignet, die die unmittelbare Umgebung der Person betreffen und die damit auf das Schlafverhalten oder den Gesundheitszustand der Person Einfluss haben können und/oder die von dem Schlafverhalten der Person beeinflusst werden (wie beispielsweise andere (zweite) Personen im gleichen Raum). Bevorzugt sind die externen Sensoren nicht physisch oder mechanisch mit dem Mundstück verbunden und/oder befinden sich nicht im Mundraum der Person, wenn das Mundstück ordnungsgemäß (wie vorgesehen) in den Mundraum der Person eigesetzt ist.

Bevorzugt weist das Gesundheitsüberwachungssystem mehrere externe Sensoren zur Messung unterschiedlicher externer Parameter auf.

Unter der unmittelbaren Umgebung der Person wird insbesondere ein geschlossener oder weitgehend geschlossener Raum verstanden, in dem die Person schläft oder sich aufhält. Dies kann beispielsweise ein Schlafzimmer oder ein Schlafsaal eines Gebäudes sein, aber auch ein Zelt oder der Innenraum eines Fahrzeugs, wie eines KFZs, eines Lastwagens, eines Campingwagens oder Wohnwagens, ein Schlafabteil in einem Zug oder eine Passagierkabine eines Flugzeugs.

Unter einem oralen Parameter sind vorliegend Messgrößen zu verstehen, die innerhalb eines Mundraums einer Person messbar sind, wie beispielsweise und erfindungsgemäß bevorzugt ein Status der Zunge (beispielsweise schlaff oder steif beziehungsweise entspannt oder angespannt, vorne (labial) oder im Rachenraum angeordnet, also die Position der Zunge im Mundraum der Person), die Temperatur im Mundraum, ein Beißdruck, eine Belastung der Kiefergelenke, eine Bewegung des Unterkiefers gegen den Oberkiefer, eine Färbung der Zunge und/oder der Mundschleimhaut, eine neuronale Aktivität zur Aktivierung von Muskeln im Mundraum oder im Bereich des Kiefers, insbesondere der Muskeln der Zunge, Geräusche, eine Feuchtigkeit, einen pH-Wert, einen Speichelfluss, Ultraschall-Reflexionen, neuronale Aktivitäten von Nervenzellen auch des zentralen Nervensystems, insbesondere im Zusammenhang mit inneren Organen der Person, einen Blutdurchfluss, eine Sauerstoffsättigung des Bluts in den Blutgefäßen im Mundraum, eine elektrische Leitfähigkeit, lokale elektrische Leitfähigkeiten, elektrische Wechselstromleitfähigkeit/Impedanz bei unterschiedlichen Wechselstrom-Frequenzen, eine Speichelzusammensetzung und Bewegungen oder die Lage des Kopfs.

Unter externen Parametern sind vorliegend Messgrößen zu verstehen, die in der Umgebung der Person, insbesondere in dem Raum, in dem die Person schläft oder aufhält, messbar sind, wie beispielsweise und erfindungsgemäß bevorzugt die Lufttemperatur, den zeitlichen Verlauf der Temperatur, den Luftdruck, die Luftfeuchtigkeit, die Zusammensetzung der Luft, die Konzentration von Staub und/oder Feinstaub in der Luft, den Sauerstoffgehalt, den CO₂-Gehalt, Daten die mit der Zeit zusammenhängen, wie beispielsweise die Uhrzeit, das Datum und/oder die Mondphasen, Geräusche im Raum, Umgebungsgeräusche, Lichtstärke und zeitlicher Verlauf der Lichtstärke im Raum, die Stärke elektromagnetischer Felder und insbesondere auch der Schlafzyklus zumindest einer zweiten Person, die im gleichen Raum schläft, sowie der zeitliche Verlauf und die zeitliche Änderung der zuvor genannten externen Parameter.

Die oralen Messdaten sind die Messdaten, die sich aus Messungen der oralen Sensoren ergeben. Dementsprechend sind die externen Messdaten diejenigen Messdaten, die sich aus Messungen der externen Sensoren ergeben. Dabei wird insbesondere der zeitliche Verlauf der oralen und externen Messdaten gespeichert, analysiert und ausgewertet.

Unter einem Mundstück ist vorliegend ein physischer Gegenstand zu verstehen, der dazu vorgesehen und dazu geeignet ist, in den Mundraum einer Person eingesetzt zu werden. Das Mundstück muss hierzu zumindest an seinen freiliegenden Oberflächen aus einem biokompatiblen Material bestehen, das auch über längere Zeit im Mundraum angeordnet werden kann, ohne dass die Materialien die Person vergiften oder andere gesundheitsschädliche Wirkungen auf die Person haben. Allergene Materialien sollten wenn möglich ebenfalls vermieden werden. Zudem soll von dem Mundstück die Atmung nicht blockiert werden können, wenn es ordnungsgemäß verwendet beziehungsweise ordnungsgemäß in den Mundraum eingesetzt wird. Bevorzugt kann das Mundstück im Mundraum der Person befestigt werden, insbesondere an im Mundraum vorhandenen Zähnen (oder auch Kronen oder Brücken oder Implantataufbauten) der Person befestigt werden, bevorzugt aufgesteckt werden. Hierzu kann vorgesehen sein, dass das Mundstück eine Passform aufweist, die eine Negativform der Zähne oder eine Negativform von einem Teilbereich mehrerer Zähne der Person aufweist. Theoretisch ist es auch denkbar, das Mundstück an der Mukosa zu befestigen oder unmittelbar an Implantaten zu befestigen.

Bevorzugt weist das Mundstück ein Befestigungsmittel zur Befestigung des Mundstücks an Zähnen und/oder der Mundschleimhaut im Mundraum der Person auf. Es kann dabei des Weiteren vorgesehen sein, dass das Befestigungsmittel eine Klemmkraft auf Strukturen im Mundraum der Person bewirkt, insbesondere auf Zähne im Mundraum der Person bewirkt, und/oder eine Adhäsion auf Oberflächen im Mundraum der Person bewirkt, insbesondere auf die feuchte Mukosa im Mundraum der Person bewirkt, wenn das Mundstück ordnungsgemäß im Mundraum der Person eingesetzt ist.

Damit die Auswertungseinrichtung zur Auswertung der oralen Messdaten des zumindest einen oralen Sensors vorgesehen sein kann, muss diese Zugriff auf Messdaten des zumindest einen oralen Sensors haben. Hierzu können der zumindest eine orale Sensor über Leiterbahnen und/oder über Funkverbindungen oder andere drahtlose Verbindungen mit der Auswertungseinrichtung verbunden sein.

Ebenso muss, damit die Auswertungseinrichtung zur Auswertung der externen Messdaten des zumindest einen externen Sensors vorgesehen sein kann, die Auswertungseinrichtung auf Messdaten des zumindest einen externen Sensors haben. Hierzu können der zumindest eine externe Sensor über Leiterbahnen und/oder über Funkverbindungen oder andere drahtlose Verbindungen mit der Auswertungseinrichtung verbunden sein.

Es kann vorgesehen sein, dass die Auswertungseinrichtung mit dem zumindest einen oralen Sensor verbunden oder verbindbar ist. Die Verbindung kann über Leiterbahnen erfolgen oder auch kabellos erfolgen. Letzteres ins insbesondere dann bevorzugt, wenn die Auswertungseinrichtung ein Teil eines externen Außengeräts ist.

Des Weiteren kann vorgesehen sein, dass die Auswertungseinrichtung mit dem zumindest einen externen Sensor verbunden oder verbindbar ist. Die Verbindung kann über Leiterbahnen erfolgen oder auch kabellos erfolgen. Theoretisch kann auch eine Auswertungseinrichtung als Teil des Mundstücks die Auswertung der Messdaten des zumindest einen externen Sensors übernehmen. Bevorzugt werden die Messdaten des zumindest einen externen Sensors jedoch von einer Auswertungseinrichtung in einem externen Außengerät übernommen.

Es kann auch vorgesehen sein, dass das Mundstück einteilig ist und an Zähnen des Oberkiefers und/oder an Zähnen des Unterkiefers der Person zu befestigen ist oder dass das Mundstück zweiteilig ist und einen Oberkieferteil aufweist, der an den Zähnen des Oberkiefers der Person zu befestigen ist, und einen Unterkieferteil aufweist, der an den Zähnen des Unterkiefers der Person zu befestigen ist, wobei bevorzugt das Oberkieferteil und das Unterkieferteil miteinander verbunden sind, besonders bevorzugt über einen motorgetriebenen Stellkörper oder über eine Positioniervorrichtung miteinander verbunden sind, wobei die Positioniervorrichtung die möglichen Stellungen des Unterkiefers gegen den Oberkiefer einschränkt.

Es kann bevorzugt vorgesehen sein, dass der zumindest eine externe Sensor zur Messung zumindest eines externen Parameters in der unmittelbaren Umgebung der Person und/oder in einem Raum, in dem die Person schläft oder sich aufhält, vorgesehen ist.

Unter einer Auswertung ist insbesondere eine computergestützte beziehungsweise rechnerisch gestützte Auswertung der digitalisierten Messdaten zu verstehen.

Bevorzugt weist das Mundstück eine eigene Auswertungseinrichtung als Teil der Auswertungseinrichtung des Gesundheitsüberwachungssystems auf, mit der orale Messdaten analysiert oder vorausgewertet werden können.

Das Mundstück weist bevorzugt einen Prozessor auf, mit dem die oralen Messdaten, die oralen Parameter und/oder die Analysen der zeitlichen Abläufe der oralen Messdaten an eine externe Auswertungseinrichtung übertragbar sind. Der Prozessor ist besonders bevorzugt in einem Kunststoffmaterial des Mundstücks eingebettet und wird mit einer Energieversorgung, wie einem elektrischen Energiespeicher, beispielsweise in Form eines Akkumulators, und/oder mit Hilfe einer Vorrichtung zum Umwandeln einer körpereigenen Energiequelle in elektrische Energie mit elektrischer Energie versorgt, wobei der elektrische Energiespeicher ganz besonders bevorzugt in einen Kunststoff des Mundstücks eingebettet ist. Die körpereigene Energiequelle kann beispielsweise auf Muskelkraft, thermischen Prozessen oder chemischen Prozessen beruhen und mit Hilfe von mit Muskelkraft getriebenen Dynamos oder mit chemischen Reaktoren oder mit thermischen Stromerzeugern angezapft werden, um die notwendige Stromversorgung sicherzustellen oder zu verbessern.

Bei erfindungsgemäßen Gesundheitsüberwachungssystemen kann bevorzugt vorgesehen sein, dass das Gesundheitsüberwachungssystem zumindest einen Aktor zur Beeinflussung des Gesundheitszustands und/oder des Schlafverhaltens der Person aufweist, wobei bevorzugt der zumindest eine Aktor ein Teil des Mundstücks ist und wobei besonders bevorzugt der zumindest eine Aktor wenigstens eine Elektrode oder einen Elektromagnet zur Beeinflussung von Nerven und/oder von Muskulatur im Mundraum aufweist und/oder der zumindest eine Aktor zumindest einen Motor zum mechanischen Verschieben des Unterkiefers gegen den Oberkiefer der Person aufweist.

Hierdurch kann mit dem Gesundheitsüberwachungssystem den Gesundheitszustand und/oder das Schlafverhalten der Person gezielt und auf Basis der Auswertungen individuell beeinflusst werden und dadurch verbessert werden. Die Elektroden oder Elektromagnete können zur Anspannung beziehungsweise Versteifung oder zur Entspannung von Muskulatur verwendet werden sowie zum Beruhigen oder Anregen von Nervenzellen.

Die Beeinflussung des Schlafverhaltens der Person bezieht sich erfindungsgemäß bevorzugt auf eine Beeinflussung des Schnarchens und/oder des Zähneknirschens der Person, insbesondere auf eine zumindest temporäre Reduzierung oder Verhinderung des Schnarchens und/oder des Zähneknirschens der Person.

Es kann auch vorgesehen sein, dass der Aktor durch ein Pelletier-Element oder ein anderes heizbares und/oder kühlbares Element realisiert wird. Mittels einer lokalen Veränderung der Temperatur im Mundraum kann eine Reaktion der Person provoziert werden und damit eine Beeinflussung des Gesundheitszustands und/oder des Schlafverhaltens der Person erfolgen. Des Weiteren kann ein Aktor auch durch ein Induktionselement realisiert werden.

Dabei kann vorgesehen sein, dass wenigstens einer des zumindest einen Aktors kein Teil des Mundstücks ist, wobei bevorzugt der wenigstens eine des zumindest einen Aktors eine Vibrationseinrichtung, eine Beleuchtung und/oder ein akustischer Signalgeber ist.

Die Vibrationseinrichtung kann bevorzugt eine Halterung zur Befestigung an einem Körperteil der Person aufweisen, so dass die Vibrationseinrichtung in Kontakt mit dem Körper der Person steht und eine Vibration oder Schwingung der Vibrationseinrichtung auf den Körper der Person übertragbar ist und dadurch den Gesundheitszustand und/oder das Schlafverhalten der Person, insbesondere die Atmung oder das Knirschen der Zähne der Person (Bruxismus), zu beeinflussen. Die Beleuchtung kann vorzugsweise in einer Schlafbrille angeordnet sein, so dass besonders bevorzugt gesteuert von dem Gesundheitsüberwachungssystem Licht mit einstellbarer, wechselnder und/oder unterschiedlicher Intensität und/oder in unterschiedlichen Farben beziehungsweise Frequenzen auf die geschlossenen Augen unter der Schlafbrille gestrahlt werden kann. Der akustische Signalgeber kann vorzugsweise ein Lautsprecher oder ein Ohrhörer sein. Auch diese Maßnahmen dienen vor allem der Beeinflussung des Schlafverhaltens der Person. Es ist auch möglich, das Licht und/oder akustische Signale in dem Raum zu steuern. Die Beleuchtung kann also auch eine Raumbeleuchtung sein und der akustische Signalgeber ein Lautsprecher im Raum.

Es ist auch möglich, dass der zumindest eine Aktor Teil des Mundstücks ist und eine Vibrationseinrichtung ist. Die Vibrationen können auch im Mundraum zur Beeinflussung des Schlafverhaltens der Person verwendet und angewendet werden.

Mit Hilfe der Aktoren kann das Gesundheitsüberwachungssystem Einfluss auf den Gesundheitszustand und/oder das Schlafverhalten und den Schlafzyklus der Person nehmen und dadurch den Schlaf der Person aktiv und individuell beeinflussen.

Des Weiteren kann vorgesehen sein, dass der zumindest eine Aktor mit einer Steuerung des Gesundheitsüberwachungssystems verbunden ist, wobei die Steuerung Zugriff auf die Analyse(n) der Auswertungseinrichtung hat und die Steuerung dazu programmiert ist, den zumindest einen Aktor in Abhängigkeit von der Analyse oder den Analysen der Auswertungseinrichtung zu steuern.

Hierdurch wird eine gezielte Beeinflussung des Gesundheitszustands und/oder des Schlafs und/oder der Schlafzustände der Person auf Basis der erkannten Schlafmuster ermöglicht. Auf diese Weise kann mit dem Gesundheitsüberwachungssystem die Gesundheit, ein gesundheitsförderlicher Schlaf gefördert oder ermöglicht werden und/oder die Störung zumindest einer zweiten Person durch Schlafgeräusche, insbesondere durch Schnarchen und/oder Bruxismus der Person reduziert werden.

Ferner kann vorgesehen sein, dass die Person eine erste Person ist und das Gesundheitsüberwachungssystem wenigstens einen externen Schlafüberwachungssensor zur Bestimmung einer Schlafphase zumindest einer zweiten Person in der Umgebung der ersten Person als einen des zumindest einen externen Sensors aufweist, wobei die Steuerung derart programmiert oder ausgestaltet ist, dass der zumindest eine Aktor in Abhängigkeit von dem Schlafzyklus der zumindest einen zweiten Person gesteuert wird, wobei bevorzugt der zumindest eine Aktor nicht betrieben wird, wenn sich die zumindest eine zweite Person in einer Tiefschlafphase befindet oder in einer Schlafphase befindet, in der Schnarchen oder Knirschen normalerweise als nicht störend empfunden wird, und wobei bevorzugt die Analyse des Schlafverhaltens der ersten Person und/oder eine Beeinflussung des Schlafverhaltens der ersten Person, insbesondere des Schnarchens der Person, mit dem zumindest einen Aktor in Abhängigkeit von der erkannten Schlafphase der wenigstens einen zweiten Person erfolgt.

Hierdurch wird es ermöglicht, den Eingriff auf das Schlafverhalten der (ersten) Person nur dann vorzunehmen, wenn die zumindest eine zweite Person gestört werden könnte. Dadurch können unnötige Eingriffe in das Schlafverhalten der ersten Person vermieden werden. Hierdurch werden die Nebenwirkungen des Gesundheitsüberwachungssystems reduziert. Eine unnötige Aktivierung oder nicht hilfreiche des zumindest einen Aktors kann so vermieden werden. Die Steuerung kann so nach Bedarf und Notwendigkeit jeder zweiten Person erfolgen, die sich beispielsweise durch Schnarchen gestört fühlen könnte. Dabei kann der zumindest eine Aktor zur Beeinflussung des Gesundheitszustands und/oder des Schlafverhaltens der ersten Person zusätzlich zur Abhängigkeit von der Schlafphase der zumindest einen zweiten Person auch in Abhängigkeit von der Lautstärke des Schnarchens oder Knirschens steuerbar sein (die mit einem der oralen Sensoren oder bevorzugt mit einem der externen Sensoren messbar ist), beziehungsweise gesteuert werden, wobei bevorzugt bei Überschreiten einer Grenzlautstärke der Aktor aktivierbar ist. Die Grenzlaustärke ist dann ein Eingriffsgrenzwert, ab dem eine Maßnahme des Gesundheitsüberwachungssystems erfolgt. Besonders bevorzugt wird die Grenzlautstärke in Abhängigkeit von der Tiefe des Schlafs der zumindest einen zweiten Person gewählt wird, wobei je tiefer der Schlaf der zumindest einen zweiten Person ist, desto höher die Grenzlautstärke des Schnarchens erlaubt wird, bevor der Aktor zur Beeinflussung des Schlafverhaltens der ersten Person aktiviert wird.

Es kann auch vorgesehen sein, dass in einer Steuerung des Gesundheitsüberwachungssystems zumindest ein Eingriffsgrenzwert gespeichert ist, der sich auf orale oder externe Parameter bezieht oder auf davon abgeleitete Größen, wobei die Steuerung ab dem Überschreiten des zumindest einen Eingriffsgrenzwerts zumindest einen des zumindest einen Aktors zur Beeinflussung des Gesundheitszustands und/oder des Schlafverhaltens der Person, insbesondere des Schnarchens der Person, aktiviert, wobei bevorzugt die der zumindest eine Eingriffsgrenzwert eine Variable ist, die von zumindest einem weiteren oralen oder externen Parameter abhängt oder von zumindest einer daraus abgeleiteten Größe, und/oder der zumindest eine Eingriffsgrenzwert in Abhängigkeit von der Analyse oder einer rechnerischen Auswertung des Gesundheitszustands und/oder des Schlafverhaltens der Person, insbesondere des Schnarchens der Person, und/oder durch manuelle Einstellung veränderbar ist.

Hierdurch kann eine Optimierung oder eine Selbstoptimierung durch das Gesundheitsüberwachungssystem erreicht werden.

Eingriffsgrenzwerte wie die Grenzlautstärke sind erfindungsgemäß bevorzugt veränderbar, insbesondere manuell einstellbar und/oder aufgrund einer Auswertung des Gesundheitszustands und/oder des Schlafverhaltens der ersten Person von der Steuerung des Gesundheitsüberwachungssystems einstellbar.

Es kann ferner vorgesehen sein, dass im Fall der Überschreitung einer vordefinierten oder einstellbaren Zeitspanne von mit der Analyse festgestellten Atemaussetzern unabhängig von dem Schlafzyklus der zumindest einen zweiten Person der zumindest eine Aktor oder wenigstens einer des zumindest einen Aktors betrieben wird. Hierdurch können Gesundheitsrisiken oder gesundheitliche Einschränkungen der (ersten) Person reduziert werden.

Des Weiteren kann vorgesehen sein, dass die Person eine erste Person ist und wenigstens ein erster externer Sensor des zumindest einen externen Sensors zur Messung eines Schlafzyklus wenigstens einer zweiten Person in der Umgebung der ersten Person ein Teil des Gesundheitsüberwachungssystems ist, wobei bevorzugt die Analyse des Schlafverhaltens der ersten Person und/oder eine Beeinflussung des Schlafverhaltens der ersten Person, insbesondere des Schnarchens der ersten Person, mit dem zumindest einen Aktor in Abhängigkeit von dem Schlafzyklus der wenigstens einen zweiten Person erfolgt.

Hierdurch wird es ermöglicht, den Eingriff auf das Schlafverhalten der (ersten) Person nur dann vorzunehmen, wenn die wenigstens eine zweite Person gestört werden könnte. Dadurch können unnötige Eingriffe in das Schlafverhalten vermieden werden. Hierdurch werden die Eingriffe und Nebenwirkungen durch gesteuerte Aktionen durch das Gesundheitsüberwachungssystems reduziert. Die Steuerung kann so nach Bedarf und Notwendigkeit jeder Person erfolgen, die sich beispielsweise durch Schnarchen oder Zähneknirschen gestört fühlen könnte.

Bevorzugt kann auch vorgesehen sein, dass der zumindest eine externe Sensor ausgewählt ist aus der Liste: Temperatursensor, Luftfeuchtigkeitssensor, Luftdrucksensor, Helligkeitssensor, Lautstärke-Sensor, Mikrofon, Chronometer, Chronometer zur Uhrzeit-Bestimmung, Chronometer zur Kalendertag-Bestimmung, Chronometer zur Mondstands-Bestimmung, Schlafzyklus-Messeinrichtung zur Bestimmung des Schlafzyklus wenigstens einer zweiten Person im gleichen Raum oder in der Umgebung, Bewegungssensor, Helligkeitssensor, Sensor zur Messung der Sauerstoffkonzentration, der Staubkonzentration und/oder der CO2-Konzentration in der Luft, Timer, wie zum Beispiel Wecker, Kamera zur Bestimmung der Position und Lage der Person, wobei bevorzugt der zumindest eine externe Sensor ein Temperatursensor, eine Uhr und/oder einen Luftdrucksensor ist.

Mit diesen Sensoren kann eine Überwachung von externen physikalischen Parametern der Umgebung erfolgen, die auf den Gesundheitszustand oder den Schlaf beziehungsweise das Schlafverhalten der Person Einfluss nehmen. Durch die Verwendung dieser externen Sensoren kann die Wechselwirkung der äußeren Einflüsse mit dem Gesundheitszustand oder dem Schlafverhalten und den oralen Parametern festgestellt und gegebenenfalls darauf reagiert werden.

Des Weiteren kann vorgesehen sein, dass der zumindest eine orale Sensor ausgewählt ist aus der Liste: Lautstärkesensor, Bisskraftsensor, Bewegungssensor zur Messung der Bewegung des Unterkiefers gegen die Oberkiefer, Temperatursensor, Feuchtigkeitssensor, Puls-Sauerstoffsensor, Vibrations- und Luftstromsensor, pH-Sensor, Lagesensor, Ultraschallsensor, Druckplattensensor, Pulslaufzeitsensor, Sensor zur Bestimmung der Färbung der Zunge und/oder der Mundschleimhäute, Atemalkoholsensor, Blutalkoholsensor, Elektromyographie-Sensor (EMG), Elektrokardiographie-Sensor (EKG), Elektroenzephalographie-Sensor (EEG), Milchsäure-Sensor, nichtinvasiver systolischer/diastolischer Blutdrucksensor, Blutzuckersensor, Zungensensor zur Bestimmung der Lage der Zunge im Mundraum, Oral-Neuro-Sensor zur Messung der neuronalen Aktivität von Zahnnerven einzelner Zähne der Person, wobei bevorzugt der zumindest eine orale Sensor einen Bisskraftsensor, einen Zungensensor zur Bestimmung der Lage der Zunge im Mundraum, einen Blutdrucksensor und/oder einen Lautstärkesensor aufweist.

Diese oralen Sensoren sind besonders gut dazu geeignet, den Gesundheitszustand oder den Schlaf beziehungsweise das Schlafverhalten der Person zu überwachen und zusammen mit den externen Einflüssen der externen Parameter deren Einfluss auf den Gesundheitszustand oder den Schlaf beziehungsweise das Schlafverhalten der Person festzustellen und gegebenenfalls Einfluss zu nehmen und auch, um die Wirkung der Aktivierung von Aktoren zu quantifizieren.

Bei einer Messung der Färbung der Zunge wird bevorzugt zwischen der Messung des Zungenkörpers und des Zungenbelags unterschieden. Das normale Zungenbild wird von den Jahreszeiten und dem Klima beeinflusst, die als externe Parameter zur Analyse vorhanden sind und daher erfindungsgemäß bei der Analyse berücksichtigt werden können. So kann im Sommer der Zungenbelag dicker ausfallen und die Zunge bei geringer Luftfeuchtigkeit und im Herbst etwas trockener als normal sein. Erfindungsgemäß werden solche Zusammenhänge und Einflüsse von externen Parametern bei der Analyse der Messungen von oralen Parametern berücksichtigt.

Gemäß einer Weiterbildung der vorliegenden Erfindung kann vorgesehen sein, dass die Auswertungseinrichtung innerhalb des Mundstücks und/oder außerhalb des Mundstücks in einem vom Mundstück separaten Gehäuse angeordnet ist, wobei bevorzugt die Auswertungseinrichtung zweiteilig ist und zumindest ein erster Teil der Auswertungseinrichtung zur Auswertung von den gesamten oder von Teilen der oralen Messdaten des zumindest einen oralen Sensors innerhalb des Mundstücks angeordnet ist und ein zweiter Teil der Auswertungseinrichtung zur Auswertung der externen Messdaten des zumindest einen externen Sensors sowie von gegebenenfalls nicht innerhalb des Mundstücks ausgewerteten oralen Messdaten des zumindest einen oralen Sensors in dem separaten Gehäuse angeordnet ist oder die gesamte Auswertungseinrichtung in dem separaten Gehäuse angeordnet ist.

Hierdurch kann zumindest ein Teil der Auswertung, insbesondere ein Teil mit hohem Rechenaufwand außerhalb des Mundstücks durchgeführt werden, wo Energie für die Rechenleistung leichter verfügbar ist. Zudem können insbesondere vorhandene externe Auswertungseinrichtungen, wie geeignet programmierte Smartphones oder Computer genutzt werden, die gegebenenfalls ohnehin vorhanden sind und die auch zum Anzeigen und/oder Übertragen der Analysen verwendet werden können.

Ferner kann vorgesehen sein, dass die Auswertungseinrichtung und gegebenenfalls die Steuerung durch eine App auf einem Smartphone oder durch ein Programm auf einem Computer oder einer anderen Recheneinheit realisiert ist.

Hierdurch kann der notwendige Ressourcenaufwand für die Auswertungseinrichtung reduziert werden, indem vorhandene Ressourcen mit hoher Rechenleistung genutzt werden. Zudem können Smartphones und Computer üblicherweise auch gleich zur Darstellung der Analysen und Daten auf einem Bildschirm sowie zur Übertragung der Daten an einen Arzt oder ein Schlaflabor genutzt werden.

Es kann auch vorgesehen sein, dass die Auswertungseinrichtung, der zumindest eine orale Sensor und der zumindest eine externe Sensor sowie gegebenenfalls die Steuerung über elektrische Kontaktierungen, wie Kabel oder Leiterverbindung, und/oder über Drahtlosverbindungen, wie Funkverbindungen, Bluetooth oder WLAN, miteinander verbunden sind, wobei bevorzugt zumindest die Auswertungseinrichtung oder ein außerhalb des Mundstücks angeordneter Teil der Auswertungseinrichtung und der zumindest eine orale Sensor über Drahtlosverbindungen, wie Funkverbindungen, Bluetooth oder WLAN, miteinander verbunden sind.

Hierdurch wird eine problemlose und für den Anwender möglichst wenig als störend empfundene Übertragung der Daten der oralen und externen Sensoren, der Steuerung und der Auswertungseinrichtung ermöglicht.

Auch kann vorgesehen sein, dass das Mundstück eine befüllbare Medikamentenabgabeeinrichtung als Aktor aufweist, wobei die Medikamentenabgabeeinrichtung von einer Steuerung des Gesundheitsüberwachungssystems in Abhängigkeit der Analyse der Auswertungseinrichtung zur Abgabe von Medikamenten aus der Medikamentenabgabeeinrichtung steuerbar ist.

Auf diese Weise ist es möglich, den Gesundheitszustand oder das Schlafverhalten der Person durch eine Abgabe von Medikamenten zu beeinflussen oder ein bestimmtes Medikament abhängig von einem anhand der oralen Parameter festgestellten gesundheitlichen Problem oder in Abhängigkeit von der Zeit zu verabreichen. Die Wahl und die Dosierung des Medikaments sollten durch einen Arzt erfolgen und die verwendeten Medikamente müssen für eine orale Anwendung geeignet sein.

Mit der Medikamentenabgabeeinrichtung können auch andere Substanzen als Medikamente abgegeben werden. Beispielsweise kann ein Bitterstoff, eine saurer Stoff, wie Vitamin C, Zitronensaft oder verdünnte Zitronensäure aus der Medikamentenabgabeeinrichtung freigesetzt werden, insbesondere im Bereich der Sauer-Rezeptoren an der Zunge, um durch die Reaktion auf den sauren Geschmack eine Versteifung der Zungenmuskulatur und/oder anderer Muskeln im Mundraum zu bewirken und dadurch beispielsweise ein Schnarchen zu verhindern oder zu beenden. Die Medikamentenabgabeeinrichtung kann folglich auch nach Art eines Aktors zur Beeinflussung des Schlafverhaltens der Person verwendet werden.

Alternativ zur Medikamentenabgabeeinrichtung am Mundstück kann auch ein steuerbares externes Pflaster oder eine von extern steuerbare Kapsel im Magen oder im Körper der Person angeordnet sein, wobei die Steuerung des Gesundheitsüberwachungssystems mittels eines Funksignals eine Wirkstofffreigabe aus dem externen Pflaster oder der Kapsel an den Körper der Person steuern kann. Bevorzugt erfolgt die Wirkstofffreigabe in Reaktion auf die Auswertung und Analyse der oralen und externen Parameter, besonders bevorzugt in Abhängigkeit von deren zeitlichem Verlauf.

Des Weiteren kann vorgesehen sein, dass das Mundstück eine an die Mundraumsituation der Person individuell angepasste Diagnoseschiene, Aktionsschiene, Aufbissschiene oder Zahnspange aufweist, wobei bevorzugt die Diagnoseschiene, Aktionsschiene oder Aufbissschiene im eingesetzten Zustand flächenbündig an den okklusalen Seiten von Zähnen des Oberkiefers und/oder des Unterkiefers der Person anliegt oder die Zahnspange flächenbündig an Bereichen der Zähne und/oder an Bereichen der Mukosa in der Mundhöhle der Person anliegt.

Hierdurch kann eine zur Behandlung von Bruxismus eingesetzte Aufbissschiene oder eine zur kieferorthopädischen Behandlung notwendige Zahnspange genutzt werden, um diese gleichzeitig als Gesundheitsüberwachungssystem einzusetzen. Da die Person diese üblicherweise ohnehin trägt, wird die Verwendung eines solchen Gesundheitsüberwachungssystems dann nicht oder kaum als zusätzliche Belastung oder Störung empfunden.

Ferner kann vorgesehen sein, dass das Mundstück im eingesetzten Zustand zumindest die koronalen Enden der Zähne des Unterkiefers und/oder des Oberkiefers des Patienten vollständig abdeckt, bevorzugt des Unterkiefers des Patienten vollständig abdeckt.

Hierdurch kann eine besonders stabile Befestigung und damit Positionierung des Mundstücks des Gesundheitsüberwachungssystems im Mundraum gewährleistet werden. Zudem kann das Mundstück auf diese Weise auch die schädlichen Auswirkungen eines Bruxismus der Person verhindern oder mindern.

Gemäß einer Weiterbildung kann vorgesehen sein, dass das Gesundheitsüberwachungssystem eine lernfähige künstliche Intelligenz aufweist, wobei die lernfähige künstliche Intelligenz mit der Auswertungseinrichtung verbunden ist und zur Erkennung von Schlafmustern und/oder von Schlafstörungen in Abhängigkeit der oralen Messdaten und der externen Messdaten oder von deren Analysen eingerichtet ist, wobei bevorzugt die künstliche Intelligenz zur Steuerung zumindest eines Aktors des Gesundheitsüberwachungssystems zur Beeinflussung des Gesundheitszustands und/oder des Schlafverhaltens der Person in Abhängigkeit von den erkannten Mustern, Schlafmustern und/oder von den erkannten Schlafstörungen eingerichtet ist, besonders bevorzugt in Abhängigkeit von den zuvor erlernten personenspezifischen oralen Messdaten und externen Messdaten oder von deren Analysen, bei denen bestimmte Muster, Schlafmuster und/oder Schlafstörungen erkannt wurden.

Hierdurch kann eine individualisierte Erkennung von personenspezifischen Mustern, Schlafmustern und/oder Schlafzuständen der Person erfolgen. Zudem können so schwer erkennbare oder bisher unbekannte Muster erkannt werden und dadurch eine Verbesserung des Erkennens und gegebenenfalls des Behandelns von kritischen Schlafzuständen und Gesundheitsproblemen der Person erreicht werden.

Es kann ferner vorgesehen sein, dass das Gesundheitsüberwachungssystem eine Anzeigeeinrichtung zur Anzeige der Analyse des Gesundheitszustands und/oder des Schlafverhaltens aufweist und/oder das Gesundheitsüberwachungssystem eine Datenübertragungseinrichtung zur Übertragung von Daten der Analyse des Gesundheitszustands und/oder des Schlafverhaltens an einen externen Computer oder ein externes Smartphone aufweist.

Hierdurch wird es der Person, dem behandelnden Arzt und/oder dem Schlaflabor ermöglicht, Einsicht in die Analysen und Messdaten zu nehmen, um basierend darauf eine Behandlung oder andere Maßnahmen zur Förderung eines gesunden Schlafs oder zur Gesundheitsverbesserung der Person ergreifen zu können. Insbesondere können auch Eingriffsgrenzwerte basierend auf den Analysen ergänzt, verändert oder angepasst werden oder die Abhängigkeit der Eingriffsgrenzwerte von anderen oralen und/oder externen Parametern angepasst werden oder neue Abhängigkeiten der Eingriffsgrenzwerte von anderen oralen und/oder externen Parametern definiert werden.

Des Weiteren kann vorgesehen sein, dass das Mundstück individuell an den Mundraum der Person angepasst ist, wobei bevorzugt zumindest eine äußere Form des Mundstücks zur Verbindung mit einer Oberfläche im Mundraum der Person, insbesondere zur Verbindung mit koronalen Enden der Zähne der Person, aus einem biokompatiblen Material 3D-gedruckt ist, besonders bevorzugt aus einem Polyamid 12-Material oder Poly-Laurylactam 3D-gedruckt ist.

Hierdurch wird eine besonders stabile Befestigung des Mundstücks im Mundraum der Person und eine besonders genaue Positionierung des Mundstücks, sowie daran angeordneter oraler Sensoren und/oder Aktoren im Mundraum erreicht.

Ferner kann vorgesehen sein, dass ein Teil des Mundstücks mit intelligenten Materialien, wie zum Beispiel mit Shape-Memory-Effekt, gefertigt ist.

Es kann auch vorgesehen sein, dass Teile des Mundstücks aus zwei oder mehr unterschiedlichen Kunststoffen 3D-gedruckt und/oder 3D-gefräst sind, wobei der zumindest eine orale Sensor und bevorzugt auch zumindest ein Teil der Analyseeinrichtung zumindest bereichsweise in die zwei unterschiedlichen Kunststoffe eingebettet sind.

Das Mundstück beziehungsweise eine Diagnoseschiene / Aktionsschiene oder Zahnschiene des Mundstücks kann 3D-gedruckt, 3D-gefräst, gegossen oder gestreut werden. Streuen ist ein zur Herstellung von Zahnspangen gebräuchliches Herstellungsverfahren, das Zahntechnikern bekannt ist. Wichtig ist, dass die zur Herstellung des Mundstücks verwendeten Materialien oder zumindest die mit der Mundschleimhaut in Kontakt kommenden Materialien des Mundstücks aus einem biokompatiblen Material bestehen.

Es kann auch vorgesehen sein, dass wenigstens einer des zumindest einen oralen Sensors ein gedruckter oraler Sensor ist, bevorzugt mit einer Drucktechnik oder mit 3D-Druck auf einen Kunststoff des Mundstücks gedruckt ist. Auch kann vorgesehen sein, dass wenigstens ein Aktor des zumindest einen Aktors gedruckt ist, bevorzugt mit einer Drucktechnik oder mit 3D-Druck auf einen Kunststoff des Mundstücks gedruckt ist.

Bevorzugte Gesundheitsüberwachungssystem können sich dadurch auszeichnen, dass der zumindest eine orale Sensor zumindest bereichsweise in zumindest einen Kunststoff des Mundstücks eingebettet ist, wobei bevorzugt ein erster Teil der Auswertungseinrichtung zumindest in den zumindest einen Kunststoff des Mundstücks eingebettet ist und ein zweiter Teil der Auswertungseinrichtung in einem von dem Mundstück separaten Gehäuse des Gesundheitsüberwachungssystems angeordnet ist.

Hierdurch kann eine stabile Verankerung und ein Schutz von empfindlichen Teilen der Sensoren und der Auswertungseinrichtung gewährleistet werden. Das Gesundheitsüberwachungssystem wird hierdurch robuster, haltbarer und weniger störanfällig.

Des Weiteren kann vorgesehen sein, dass der zeitliche Verlauf der muskulären Spannung der Zunge oder der Versteifung der Zungenmuskulatur und/oder die Atemfrequenz der Person und deren Gleichmäßigkeit mit Hilfe von wenigstens einem des zumindest einen oralen Sensors überwachbar ist und bevorzugt von dem Gesundheitsüberwachungssystem elektronisch speicherbar und/oder von der Auswertungseinrichtung analysierbar ist.

Hierdurch können die Ursache für Apnoe, Schnarchen und das Schnarchen selbst erkannt, analysiert und gegebenenfalls behandelt werden. Auf diese Weise kann der Schlaf der Person oder der zumindest einen zweiten Person verbessert werden oder Maßnahmen ergriffen werden, um deren Schlaf zu verbessern.

Auch kann vorgesehen sein, dass im Mundstück ein elektrischer Energiespeicher enthalten ist, wobei mit dem elektrischen Energiespeicher der zumindest eine orale Sensor und, falls im oder am Mundstück vorhanden, der zumindest eine Aktor, eine Steuerung, ein Teil der Auswertungseinrichtung und/oder eine Datenübertragungseinrichtung mit elektrischer Energie versorgbar ist, wobei bevorzugt der elektrische Energiespeicher in zumindest einen Kunststoff des Mundstücks eingebettet ist.

Hierdurch ist eine Energieversorgung der Sensoren am Mundstück und gegebenenfalls eines Teils der Auswertungseinrichtung sichergestellt. Der elektrische Energiespeicher oder Akkumulator kann durch Einkapselung geschützt sein. Der elektrische Energiespeicher ist erfindungsgemäß bevorzugt ein Akkumulator. Ebenfalls bevorzugt kann der elektrische Energiespeicher kabellos aufgeladen werden. Es kann auch vorgesehen sein, dass der elektrische Energiespeicher durch körpereigene thermische, chemische oder mechanische Prozesse wiederaufladbar ist. Theoretisch kann als elektrischer Energiespeicher auch eine Batterie verwendet werden, bevorzugt eine austauschbare Batterie.

Es ist auch möglich, den zumindest einen oralen Sensor oder wenigstens einen des zumindest eines oralen Sensors mittels RFID auszulesen und zu betreiben.

Ferner kann vorgesehen sein, dass das Mundstück eine Schiene aufweist, wobei die Schiene auf den Zahnbogen oder auf die Mukosa des zahnlosen Kieferbogens des Oberkiefers und/oder auf den Zahnbogen oder auf die Mukosa des zahnlosen Kieferbogens des Unterkiefers der Person aufsteckbar und/oder zu befestigen ist.

Hierdurch sind eine stabile Verbindung und eine genaue Positionierung des Mundstücks im Mundraum der Person möglich. Bevorzugt ist die Schiene eine Aufbissschiene, eine Diagnoseschiene oder eine Aktionsschiene, besonders bevorzugt eine Aufbissschiene.

Gemäß einer bevorzugten Weiterbildung kann vorgesehen sein, dass das Mundstück eine Mehrzahl von Oral-Neuro-Sensoren zur Messung neuronaler Aktivitäten von Nerven im Mundraum als orale Sensoren aufweist, wobei jeder Oral-Neuro-Sensor zumindest eine Messelektrode aufweist, die mit zumindest einem der Zähne oder mit einem Zahnhals oder einer Gingiva-Linie zumindest eines der Zähne der Person oder der Gingiva oder der Mukosa oder der Zunge in unmittelbarem Kontakt stehen, wenn das Mundstück ordnungsgemäß im Mundraum des Patienten eingesetzt ist, wobei die Oral-Neuro-Sensoren bevorzugt zur Messung von Strömen oder elektrischen Feldern der Zahnnerven oder von Nerven in der Gingiva, der Mukosa oder der Zunge der Person geeignet und vorgesehen sind, wobei besonders bevorzugt jedem Zahn der Person ein Oral-Neuro-Sensor zugeordnet ist.

Diese Oral-Neuro-Sensoren ermöglichen eine oder verschiedene gezielte Überwachung(en) von neuronalen Aktivitäten einzelner Zahnnerven oder anderer Nervenzellen im Mundraum. Dies ermöglicht Rückschlüsse auf mögliche Probleme im oralen Bereich aber auch auf Probleme von inneren Organen der Person, die über Nervenbahnen mit den Zahnnerven einzelner Zähne oder anderer oraler Nerven der Person gekoppelt sind. Dadurch können bestimmte Krankheitsbilder oder Probleme mit bestimmten Organen der Person erkannt werden. Durch die Bestimmung des elektrischen Widerstands an den Zahn-Nerven oder an anderen oralen Nerven können solche Rückschlüsse gezogen werden. Der Fachmann kennt gegebenenfalls diese und weitere Zusammenhänge zwischen einzelnen Zahnnerven oder anderen oralen Nervenzellen und Organen oder Körperteilen sowie andere Verfahren zur Messung und Bestimmung von Oral-Nerven-Organ-Verbindungen und Zahnnerven-Organ-Verbindungen.

Unter einer Messelektrode eines Oral-Neuro-Sensors wird vorliegend eine Elektrode verstanden, die zur Messung eines elektrischen Widerstands oder zur Aufnahme von elektrischen und/oder magnetischen Felder geeignet ist, die durch neuronale Aktivitäten im Mundraum verursacht werden.

Des Weiteren kann vorgesehen sein, dass das Mundstück zumindest einen Elektromagnet als den zumindest einen Aktor oder als einer des zumindest einen Aktors aufweist, wobei der zumindest eine Elektromagnet zum Erzeugen eines Magnetfelds im Bereich zumindest eines Nervs oder Muskels im Mundraum der Person geeignet und vorgesehen ist, wenn das Mundstück ordnungsgemäß im Mundraum des Patienten eingesetzt ist.

Auf diese Weise lassen sich Nerven und Muskeln im Mundraum beeinflussen, um den Schlaf zu verbessern und um die Zunge und/oder den Unterkiefer der Person in eine gewünschte Stellung zu bringen, um Schnarchen oder Bruxismus zu verhindern oder zu stoppen.

Ferner kann vorgesehen sein, dass das Mundstück eine Vielzahl von Ausnehmungen aufweist, insbesondere eine Vielzahl von Ausnehmungen mit gleichen inneren Oberflächen aufweist, und wenigstens ein austauschbarer Sensor des zumindest einen oralen Sensors in wenigstens einer der Ausnehmungen lösbar befestigt ist, wobei bevorzugt der wenigstens eine austauschbare Sensor eine äußere Halterung aufweist, die flächenbündig mit den Ausnehmungen des Mundstücks verbindbar ist, insbesondere mit den inneren Oberflächen der Ausnehmungen des Mundstücks verbindbar ist, wenn der wenigstens eine austauschbare Sensor in der Ausnehmung befestigt ist, wobei besonders bevorzugt das Gesundheitsüberwachungssystem eine Vielzahl von austauschbaren Sensoren aufweist, wobei die austauschbaren Sensoren äußere Halterungen mit gleichförmiger äußerer Oberfläche aufweisen, wobei die äußeren Oberflächen flächenbündig mit mehreren der Ausnehmungen des Mundstücks verbunden sind oder verbindbar sind, insbesondere mit den inneren Oberflächen der Ausnehmungen des Mundstücks verbunden sind oder verbindbar sind.

Hierdurch können zumindest einige der oralen Sensoren ausgetauscht werden und bei Bedarf in dem Mundstück individuell positioniert werden. Dadurch muss das gesamte Mundstück nicht neu gefertigt werden, falls einer der oralen Sensoren ergänzt werden soll oder anders und/oder an einer anderen Stelle in dem Mundstück positioniert werden soll.

Um die austauschbaren Sensoren variabel in den Ausnehmungen des Mundstücks positionieren zu können, kann vorgesehen sein, dass die Ausnehmungen im Mundstück konisch geformt sind und die äußeren Halterungen der austauschbaren Sensoren eine konische äußere Oberfläche aufweisen, bevorzugt konische äußere Oberflächen mit gleichen Öffnungswinkeln und unterschiedlichen Außendurchmessern aufweisen. Hierdurch kann eine variable Positionierung relativ zur äußeren Oberfläche des Mundstücks erreicht werden.

In gleicher Weise können auch zumindest einige der Aktoren als austauschbare Aktoren vorliegen die in den Ausnehmungen wechselbar angeordnet werden können. Bevorzugt haben austauschbare Aktoren und austauschbare Sensoren eine Halterung mit gleichen oder gleichartigen äußeren Oberflächen oder mit konischen äußeren Oberflächen mit gleichen Öffnungswinkeln und unterschiedlichen Außendurchmessern aufweisen.

Zumindest eine der vorgenannten Aufgaben oder eine sich aus dem Stand der Technik ergebende Aufgabe wird des Weiteren zumindest teilweise gelöst durch ein Verfahren zur Analyse eines Gesundheitszustands oder eines Schlafverhaltens einer Person oder zur Analyse und Beeinflussung des Gesundheitszustands oder des Schlafverhaltens der Person, mit den Schritten:
A) Messen eines zeitlichen Verlaufs zumindest eines oralen Parameters im Mundraum der Person und Messen eines zeitlichen Verlaufs zumindest eines externen Parameters in der Umgebung der Person;
B) rechnerische Analyse der zeitlichen Verläufe des zumindest einen oralen Parameters und des zumindest einen externen Parameters; und^
C) Identifizieren von Zusammenhängen und Mustern des Gesundheitszustands oder im Schlafverhalten und/oder von Schlafzuständen der Person anhand der rechnerischen Analyse.

Dabei kann vorgesehen sein, dass bei dem Verfahren ein zuvor genannten oder erfindungsgemäßes Gesundheitsüberwachungssystem verwendet wird.

Die Verfahren haben dann die zu dem Gesundheitsüberwachungssystem genannten Vorteile.

Des Weiteren kann vorgesehen sein, dass in einem Schritt D) ein Aktivieren zumindest eines Aktors in Abhängigkeit von den in Schritt C) identifizierten Zusammenhängen und Mustern im Gesundheitszustand, Schlafverhalten und/oder Schlafzuständen der Person erfolgt, wobei der zumindest Aktor die Person berührt, insbesondere elektrisch, magnetisch und/oder mechanisch kontaktiert, und/oder durch elektrische und/oder magnetische Felder auf Nervenzellen der Person einwirkt, wobei bevorzugt die Wirkung eines aktivierten Aktors des zumindest einen Aktors auf den Gesundheitszustand, den Schlaf oder das Schlafverhalten der Person durch die fortlaufende rechnerische Analyse des zeitlichen Verlaufs des zumindest einen oralen Parameters oder durch die fortlaufende rechnerische Analyse der zeitlichen Verläufe des zumindest einen oralen Parameters und des zumindest einen externen Parameters verfolgt wird, wobei besonders bevorzugt die Intensität des zumindest einen Aktors in Abhängigkeit von der nachverfolgten Wirkung des aktivierten Aktors modifiziert wird.

Hierdurch kann mit dem zumindest einen Aktor in Abhängigkeit von der Analyse des Schlafverhaltens der Person Einfluss auf den Gesundheitszustand und/oder das Schlafverhalten der Person genommen werden und gegebenenfalls die Wirkung des aktivierten Aktors festgestellt und gegebenenfalls angepasst werden.

Dabei kann vorgesehen sein, dass zumindest eine Elektrode zur Stimulation wenigstens eines Muskels im Mundraum, insbesondere wenigstens eines Zungenmuskels, als Aktor aktiviert wird, wenigstens ein Elektromagnet zur Beeinflussung von Nervenzellen oder Muskulatur im Bereich des Mundraums als Aktor aktiviert wird und/oder dass als Aktor ein Motor zum Verschieben des Unterkiefers gegen den Oberkiefer aktiviert wird.

Hierdurch kann ein Schnarchen oder Zähneknirschen der Person beeinflusst, reduziert oder verhindert werden, indem die muskuläre Zungenversteifung erhöht wird und/oder die Stellung des Unterkiefers gegen den Oberkiefer verändert wird.

Ferner kann vorgesehen sein, dass in einem Schritt E) ein personenspezifisches Erlernen von Zusammenhängen zwischen den analysierten zeitlichen Verläufen des zumindest einen oralen Parameters und des zumindest einen externen Parameters und Gesundheitszuständen und/oder Schlafzuständen der Person erfolgt, wobei bevorzugt die in Schritt E) aus vorherigen Messungen und/oder Schlafzyklen erlernten personenspezifischen Zusammenhänge in Schritt C) verwendet werden, um die Zusammenhänge und Muster im Gesundheitszustand und/oder Schlafverhalten und/oder die Schlafzustände der Person zu erkennen und/oder um die Aktivierung des Aktors in Schritt D) zu steuern.

Hierdurch wird eine individualisierte Analyse des Gesundheitszustands und/oder des Schlafverhaltens und gegebenenfalls auch eine Individualisierung der Beeinflussung beziehungsweise der Behandlung des Gesundheitszustands und/oder Schlafverhaltens der Person ermöglicht. Dies kann besonders bevorzugt durch Verwenden einer künstlichen Intelligenz zum Erkennen des Gesundheitszustands und/oder der Schlafzustände erreicht werden.

Es kann auch vorgesehen sein, dass aus der rechnerischen Analyse in Schritt B) zusätzlich mögliche gesundheitliche Probleme der Person abgeleitet werden und über eine Anzeigeeinrichtung angezeigt oder über eine Datenübertragungseinrichtung an einen Computer oder ein Smartphone ausgegeben werden.

Hierdurch kann eine anschließende Auswertung der Daten und Analysen durch die Person selbst, durch einen Arzt oder durch ein Schlaflabor erfolgen, um daraus Behandlungsmethoden oder Maßnahmen zur Förderung des Schlafs der Person abzuleiten und auch um das Gesundheitsüberwachungssystem selbst einzustellen und deren Regelungen anzupassen.

Des Weiteren kann vorgesehen sein, dass in Schritt C) die Analyse elektronisch gespeichert wird und/oder über eine Anzeigeeinrichtung angezeigt oder dargestellt wird und/oder über eine Datenübertragungseinrichtung an ein externes Gerät an einen Computer oder ein Smartphone zum Zweck einer nachfolgenden Diagnose durch einen Arzt ausgegeben wird.

Hierdurch kann eine anschließende Auswertung der Daten und Analysen durch die Person selbst, durch einen Arzt oder durch ein Schlaflabor erfolgen, um daraus Behandlungsmethoden oder Maßnahmen zur Förderung des Schlafs der Person abzuleiten.

Zumindest einer der der vorgenannten oder sich aus dem Stand der Technik ergebenden Nachteile wird auch gelöst durch ein Mundstück für ein erfindungsgemäßes beziehungsweise oben genanntes Gesundheitsüberwachungssystem, wobei das Mundstück zum Einsetzen in den Mundraum der Person vorgesehen ist und wobei das Mundstück zumindest einen oralen Sensor aufweist, wobei der zumindest eine orale Sensor zur Messung zumindest eines oralen Parameters im Mundraum der Person vorgesehen ist, wobei eine Auswertungseinrichtung mit dem zumindest einen oralen Sensor verbunden oder verbindbar ist, insbesondere über eine Sendereinrichtung und/oder Sender- / Empfängereinrichtung des Mundstücks kabellos verbunden oder verbindbar ist. Weitere vorteilhafte erfindungsgemäße Ausgestaltungen des erfindungsgemäßen Mundstücks sind der vorherigen Ausführungen und den Ausführungsbeispielen zu entnehmen.

Zusammengefasst betrifft die vorliegende Erfindung ein Gesundheitsüberwachungssystem zur Analyse und/oder Beeinflussung des Gesundheitszustands und/oder des Schlafverhaltens einer Person aufweisend ein Mundstück und eine Auswertungseinrichtung, zumindest einen oralen Sensor zur Messung eines oralen Parameters, wobei das Gesundheitsüberwachungssystem zumindest einen externen Sensor aufweist, der kein Teil des Mundstücks ist und der zur Messung zumindest eines externen Parameters vorgesehen ist, wobei die Auswertungseinrichtung derart programmiert ist, dass eine Analyse des Gesundheitszustands und/oder des Schlafverhaltens der Person unter Berücksichtigung sowohl der Auswertung der oralen Messdaten als auch der Auswertung der externen Messdaten erfolgt.

Der Erfindung liegt die überraschende Erkenntnis zugrunde, dass es durch die Verwendung und gemeinsame Auswertung und Analyse von oralen Parametern und externen Parametern gelingt, die Wechselwirkung zwischen externen Einflüssen und personenspezifischen körperlichen Reaktionen zu berücksichtigen, um eine umfangreichere und ganzheitlichere Analyse des Schlafverhaltens der Person bereitzustellen. Dadurch wird eine exaktere Analyse des Gesundheitszustands und/oder des Schlafverhaltens ermöglicht und es können bisher unbekannte Wechselwirkungen und Einflüsse festgestellt werden. Mittels der erfindungsgemäßen Analyse kann eine genauere Diagnose und bei Bedarf eine zielgerichtetere und schonendere Beeinflussung des Gesundheitszustands und/oder des Schlafverhaltens der Person erfolgen. Durch die gezieltere und schonendere Beeinflussung des Schlafverhaltens kann ein erholsamerer Schlaf der Person erreicht werden.

Mit der vorliegenden Erfindung wird zum ersten Mal ein ganzheitlicher Ansatz bei der Gesundheitsanalyse beziehungsweise Schlafanalyse mit einem Gesundheitsüberwachungssystemen mit Mundstück verfolgt, bei dem die Umgebungseinflüsse auf die Gesundheit und/oder den Schlaf der Person gemessen und in Korrelation mit dem Gesundheitszustand und/oder dem Schlafverhalten der Person gesetzt werden. Auf diese Weise kann beispielsweise erkannt werden, dass ein bestimmter externer Parameter aus der unmittelbaren Umgebung (wie beispielsweise die Temperatur oder die Luftfeuchtigkeit) für die Gesundheit und/oder den Schlaf der Person wesentlich störender wirken als andere externe Parameter (wie zum Beispiel die Lautstärke und die Helligkeit) und auch unter welchen Umständen sich diese Einflüsse wie stark auf die Gesundheit und/oder den Schlaf auswirken. Durch eine Darstellung oder Datenübertragung auf einen Computer oder ein Smartphone kann das Gesundheitsüberwachungssystem diese Analysen und Zusammenhänge an die Person, einen Arzt oder ein Schlaflabor weitergeben. Dadurch werden diese in die Lage versetzt, geeignete Maßnahmen zu ergreifen, um die Gesundheit und/oder den Schlaf der Person zu verbessern und gesundheitsbeeinträchtigende beziehungsweise schlafstörende Zustände zu vermeiden.

Dabei wurde im Rahmen der vorliegenden Erfindung überraschend erkannt, dass als wichtiges Kriterium, ob ein Schnarchen der (ersten) Person für zumindest eine zweite Person, die im gleichen Raum und in der unmittelbaren Umgebung schläft, als störend empfunden wird. Dies hängt maßgeblich von der Schlafphase der zumindest einen zweiten Person ab. Daher wurde erfindungsgemäß erkannt, dass es einer Beeinflussung und damit einer Störung der Schlafphase der (ersten) Person nicht bedarf, falls sich die zumindest eine zweite Person im Tiefschlaf befindet oder sich in einer Schlafphase befindet, die derart tief ist, dass die zumindest eine zweite Person üblicherweise nicht durch das Schnarchen oder Knirschen oder erst durch ein Schnarchen oder ein Knirschen ab einer höheren Lautstärke gestört werden kann. Wenn dieser externe Parameter ("störungsfreies Schnarchen der (ersten) Person ist möglich") festgestellt beziehungsweise mit einem externen Sensor gemessen und mit einer Analyse der externen Messdaten erkannt wird, dann kann auf eine Beeinflussung und dadurch Störung des Schlafs beziehungsweise des Schlafverhaltens der (ersten) Person verzichtet werden und damit ein erholsamerer Schlaf der ersten Person gewährleistet werden.

Des Weiteren können mit dem erfindungsgemäßen Gesundheitsüberwachungssystem Verschlechterungen des Gesundheitszustands und bestimmte Krankheitsbilder erkannt werden. Diese können frühzeitig erkannt und dadurch gegebenenfalls frühzeitig therapiert werden. Besonders einfache Therapien können dabei gegebenenfalls mit Hilfe von Aktoren während des Schlafs der Person vorgenommen werden. Diese zielen vor allem darauf ab, den Schlaf der Person zu verbessern und gleichzeitig gesundheitsbeeinträchtigende Schlafzustände zu vermeiden. Ferner kann es auch darum gehen, den Schlaf zumindest einer zweiten Person, wie beispielsweise einen Ehepartner, zu schonen, um eine Beeinträchtigung des Soziallebens der ersten Person zu vermeiden.

Bei einer Ausführung des Gesundheitsüberwachungssystems, bei dem im Mundstück für verschiedene Zähne und/oder Nerven im Mundraum der Person Oral-Neuro-Sensoren als orale Sensoren zur Messung neuronaler Aktivitäten einzelner Zahnnerven und/oder bestimmter Nerven in der Gingiva, der Mukosa und/oder der Zunge vorhanden sind, können zusätzliche zu den Auswirkungen des Bruxismus auch noch Rückschlüsse auf mögliche Erkrankungen oder Beeinträchtigungen von Organen der Person gezogen und derartige gesundheitliche Probleme analysiert werden. Dabei kann das Konzept der Zahn-Organ-Beziehung beziehungsweise Oral-Nerv-Organ-Beziehung genutzt werden, gemäß dem Zahnkrankheiten und neuronale Aktivitäten der Zahnnerven oder von anderen Nerven im Mundraum nicht als isolierter eigenständiger Prozess betrachtet werden und einzelne Zähne oder anderes Nervengewebe im Mundraum als in einer Wechselbeziehung zu Organen stehend betrachtet werden.

Die ganzheitliche Zahnmedizin geht davon aus, dass jeder Zahn eine bestimmte Verbindung zu einem Organ oder einem Teil des Körpers hat. Ein Problem mit einem bestimmten Zahn kann den Energiefluss in einem bestimmten Meridian beeinträchtigen, eine Stagnation verursachen und schließlich zu einer Krankheit beitragen und umgekehrt.

Ähnlich Akupunkturpunkten kann die Zahn-Organ-Beziehung beziehungsweise die Oral-Nerv-Organ-Beziehung auf Probleme bei bestimmten Organen hinweisen. Laut der traditionellen Chinesischen Medizin sind bestimmte Zahngruppen, wie die Front-, Eck-, sowie Backenzähne, in den Energiefluss des Körpers eingebunden und stehen somit in direkter Verbindung mit bestimmten Organen. Die Schneidezähne sind in einer Wechselbeziehung zu den Nieren und der Blase, die Eckzähne stehen mit der Leber, Galle sowie den Augen in Verbindung, die kleinen und großen Backenzähne stehen in einer Wechselbeziehung zum Magen- und Darmtrakt und die Weisheitszähne werden mit dem Herz und dem allgemeinen Energiehaushalt in Verbindung gebracht. Zusätzlich stehen die Zahnnerven auch mit den Gelenken und verschiedenen Teilen der Wirbelsäule in Verbindung. Weitere Zusammenhänge sind dem Fachmann anhand von Zahnmeridiankarten (Tooth-Organ Meridian Chart) bekannt und auf diese Weise für den Fachmann anwendbar. Dementsprechend kann eine erhöhte oder eine ansteigende neuronale Aktivität der Zahnnerven der jeweiligen Zahngruppen oder von einzelnen Zähnen ein Hinweis auf das Vorliegen einer Erkrankung eines bestimmten Organs oder einer Problematik mit dem Organ sein. Mit einem erfindungsgemäßen Gesundheitsüberwachungssystem und einem erfindungsgemäßen Verfahren können derartige Probleme durch die Analyse erkannt werden. Gleichzeitig kann der Einfluss auf das Schlafverhalten analysiert werden oder auch eine Hinweis über eine Anzeigeeinrichtung an die Person, den behandelnden Arzt oder ein Schlaflabor weitergeleitet werden. Im Idealfall kann zusätzlich durch eine behutsame magnetische oder elektrische Aktivierung der Zahnnerven mittels geeigneter Aktoren am Mundstück auch eine Therapierung der Probleme versucht werden.

Die Störung des Energieflusses zwischen Zähnen und Organen kann mittels Elektroakupunktur nach Dr. Reinhold Voll (EAV) erkannt und behandelt werden. Dabei werden keine Nadeln verwendet. Stattdessen soll die Messung des elektrischen Widerstands an den Akupunkturpunkten Rückschlüsse auf mögliche gesundheitliche Probleme ermöglichen. Daher wird mit der vorliegenden Erfindung auch oralen Sensoren vorgeschlagen, mit denen die elektrischen Widerstände an den oralen Akupunkturpunkten nach Dr. Voll messbar sind. Durch das Stimulieren der Therapiepunkte mittels erfindungsgemäßer Anwendung von Aktoren kann der gestörte Energiefluss wiederhergestellt werden und so gegebenenfalls und unter Umständen auch chronische Erkrankungen therapiert werden. Des Weiteren könnte auch eine Beeinflussung von Körperfunktionen, wie die Verdauung, Kopfschmerzen und Muskelverspannungen erfolgen. Andere Messverfahren zur Messung als die Messung elektrischer Widerstände sind zur Bestimmung neuronaler Aktivitäten vorstellbar. Beispielsweise könnten magnetische oder elektrische Felder im Bereich der Nervenzellen gemessen werden.

Auch wenn die Zahn-Organ-Beziehung beziehungsweise Oral-Nerv-Organ-Beziehung wissenschaftlich noch nicht belegt ist, ist es unstrittig, dass entzündete Zähne und andere neuronale Entzündungen im Mundraum einen Einfluss auf den restlichen Körper haben. Daher kann auch eine lokale verstärkte Durchblutung ein Hinweis auf tiefergehende gesundheitliche Problematiken sein.

Im Folgenden werden Ausführungsbeispiele der Erfindung anhand von drei schematisch dargestellten Figuren und einem Ablaufdiagramm erläutert, ohne jedoch dabei die Erfindung zu beschränken. Dabei zeigt:
Figur 1: eine schematische Darstellung eines ersten erfindungsgemäßen Gesundheitsüberwachungssystems mit einem Mundstück und zwei externen Geräten;
Figur 2: eine schematische Querschnittansicht eines alternativen Mundstücks eines zweiten erfindungsgemäßen Gesundheitsüberwachungssystems im Mundraum einer Person;
Figur 3: eine schematische, transparente Aufsicht auf ein weiteres alternatives Mundstück eines dritten erfindungsgemäßen Gesundheitsüberwachungssystems, wobei das Mundstück im Mundraum einer Person eingesetzt ist; und
Figur 4: den Ablauf eines erfindungsgemäßen Verfahrens zum Messen und Beeinflussen des Gesundheitszustands und/oder des Schlafverhaltens einer Person.

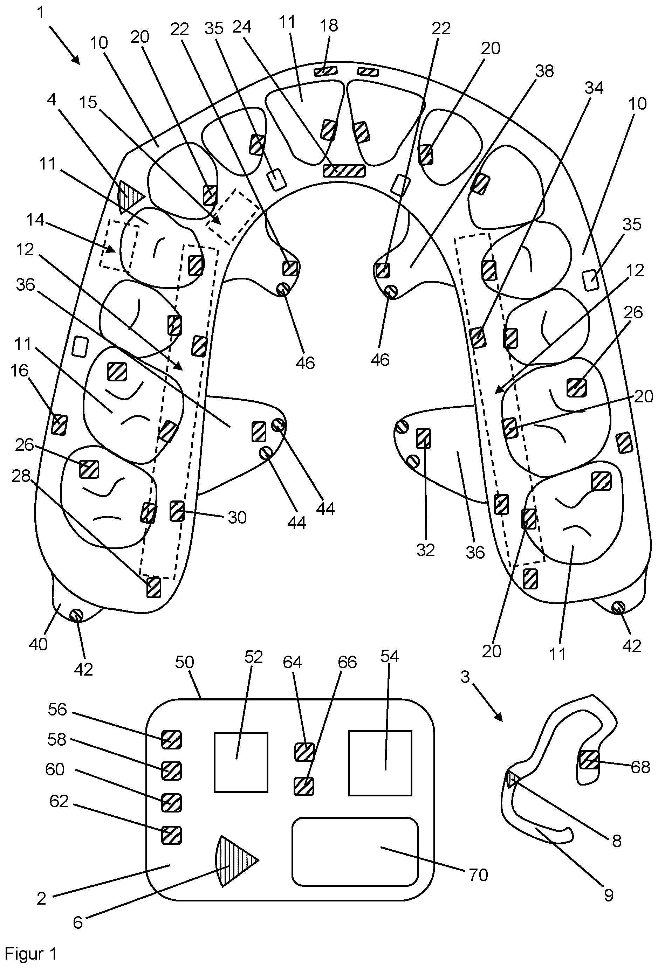

Figur 1 zeigt in einer schematischen Darstellung ein erstes erfindungsgemäßes Gesundheitsüberwachungssystem zur Analyse des Gesundheitszustands und/oder eines Schlafverhaltens einer (ersten) Person (nicht dargestellt) oder zur Analyse und Beeinflussung der Gesundheit und/oder des Schlafverhaltens einer (ersten) Person. Das Gesundheitsüberwachungssystem weist ein Mundstück 1, ein externes Außengerät 2 und vorliegend auch ein externes Überwachungsgerät 3 auf. Das externe Überwachungsgerät 3 ist optional. Das Mundstück 1 ist zum Einsetzen in den Mundraum der Person geeignet und vorgesehen. Insbesondere ist das Mundstück 1 zum Befestigen an einer Zahnleiste eines Unterkiefers der Person geeignet und vorgesehen. Das externe Außengerät 2 ist dafür vorgesehen, in der Umgebung der Person (im gleichen Raum) angeordnet zu werden, in dem die Person schläft oder sich aufhält und während die Person schläft oder wacht. Das externe Überwachungsgerät 3 dient der Überwachung des Schlafzustands einer zweiten Person, die sich in dem gleichen Raum (in der gleichen Umgebung) aufhält wie die erste Person, oder zur Überwachung des Schlafzustands mehrerer zweiter Personen, die sich in dem gleichen Raum (in der gleichen Umgebung) aufhalten beziehungsweise schlafen wie die erste Person.

Das Mundstück 1, das externe Außengerät 2 und das externe Überwachungsgerät 3 weisen bevorzugt alle jeweils eine Sender- / Empfängereinrichtung 4, 6, 8 auf, über die das Mundstück 1 zum Austausch von Daten mit dem externen Außengerät 2 und über die das externe Überwachungsgerät 3 zum Austausch von Daten mit dem externen Außengerät 2 verbunden ist beziehungsweise bei Bedarf verbunden werden kann. Bevorzugt wird hierzu eine Drahtlos-Verbindung, wie eine Funkverbindung, Bluetooth oder WLAN genutzt, um einen Datenaustausch zwischen dem Mundstück 1 und dem externen Außengerät 2 sowie zwischen dem externen Überwachungsgerät 3 und dem externen Außengerät 2 zu ermöglichen. Die Datenverbindung über die Sender- / Empfängereinrichtung 4, 6, 8 muss nicht immer beziehungsweise nicht kontinuierlich aktiv sein, sondern kann temporär zum Austausch von Daten genutzt werden.

Das externe Überwachungsgerät 3 kann ein Befestigungsband 9 zur Befestigung des externen Überwachungsgeräts 3 an der zweiten Person aufweisen oder auch irgendein anderes Befestigungsmittel zur Befestigung des externen Überwachungsgeräts an der zweiten Person. Es kann auch vorgesehen sein, dass das Gesundheitsüberwachungssystem mehrere solcher externen Überwachungsgeräte 3 aufweist, die zur Überwachung des Gesundheitszustands und/oder des Schlafs von mehreren zweiten Personen verwendet werden können, beispielsweise mehrere zweite Personen, die zusammen mit der ersten Person in einem Schlafsaal, einem Zelt oder einem Abteil übernachten.

Das Mundstück 1 kann bevorzugt eine anatomisch individuell angepasste Diagnoseschiene / Aktionsschiene 10 mit Zahnaufnahmevertiefungen 11 aufweisen. Bevorzugt ist die Diagnoseschiene / Aktionsschiene 10 eine Aufbissschiene. Theoretisch kann das Mundstück 1 aber auch ohne Diagnoseschiene / Aktionsschiene 10 realisiert werden und in seiner äußeren Form dennoch an die Oberfläche des Mundraums der Person angepasst sein. Die okklusalen Seiten beziehungsweise die okklusalen Oberflächen der Zähne der Person werden bei der Ausführung mit Diagnoseschiene / Aktionsschiene 10 bevorzugt formschlüssig und flächenbündig in den Zahnaufnahmevertiefungen 11 aufgenommen. Auf diese Weise kann die Diagnoseschiene / Aktionsschiene 10 und damit das gesamte Mundstück 1 von den Zähnen im Mundraum der Person gehalten werden. Bevorzugt liegt das Mundstück 1 auch noch zusätzlich an anderen Bereichen des Mundraums der Person (auch auf der Mukosa) an, wenn das Mundstück 1 ordnungsgemäß im Mundraum der Person eingesetzt ist. Die Diagnoseschiene / Aktionsschiene 10 kann gleichzeitig die Zähne der Person während des Schlafs vor einem Abrieb durch Bruxismus schützen. Bevorzugt wird die Diagnoseschiene / Aktionsschiene 10 aus einem Kunststoff, insbesondere auch einem Polyamid-12-Material oder aus Poly-Laurylactam, basierend auf einem individuellen 3D-Modell eines Teils des Mundraums der Person 3D-gedruckt, um eine besonders stabile und abriebfeste Diagnoseschiene / Aktionsschiene 10 zur Verfügung zu stellen. Neben dem 3D-Druck kann die Diagnoseschiene / Aktionsschiene 10 auch 3D-gefräst werden oder durch Spritzgussverfahren oder mit Streuverfahren hergestellt werden. Die Diagnoseschiene / Aktionsschiene 10 oder zumindest alle außenliegenden Teile des Mundstücks 1 werden aus biokompatiblen Materialien hergestellt.

Eingebettet in das Material des Mundstücks 1 oder der Diagnoseschiene / Aktionsschiene 10 kann ein elektrischer Energiespeicher 12 in Form eines Akkumulators angeordnet sein. Der elektrische Energiespeicher 12 ist in Figur 1 zweiteilig und auf beiden Seiten des Zahnbogens angeordnet. Der elektrische Energiespeicher 12 ist bevorzugt drahtlos aufladbar (beispielsweise mit einem Ladegerät analog einer Handyschale) und dient der Spannungsversorgung der Sender- / Empfängereinrichtung 4 des Mundstücks 1 sowie einer Auswertungseinrichtung 14 und einer Steuerung 15 in Form eines Prozessors. Der elektrische Energiespeicher 12 kann mit Hilfe einer Vorrichtung zum Umwandeln einer körpereigenen Energiequelle in elektrische Energie (nicht gezeigt) mit elektrischer Energie aufgeladen werden. Es ist auch möglich, die Sender- / Empfängereinrichtung 4 des Mundstücks 1, die Auswertungseinrichtung 14 und die Steuerung 15 in Form eines Prozessors mit elektrischer Spannung der Vorrichtung zum Umwandeln einer körpereigenen Energiequelle in elektrische Energie zu versorgen. Die körpereigene Energiequelle kann beispielsweise auf Muskelkraft, thermischen Prozessen oder chemischen Prozessen beruhen und mit Hilfe von mit Muskelkraft getriebenen Dynamos oder mit chemischen Reaktoren oder mit thermischen Stromerzeugern angezapft werden, um die notwendige Stromversorgung sicherzustellen oder zu verbessern. Die Auswertungseinrichtung 14 und die Steuerung 15 können ebenfalls in ein Kunststoffmaterial des Mundstücks 1 oder der Diagnoseschiene / Aktionsschiene 10 eingebettet sein. Die Auswertungseinrichtung 14 und die Steuerung 15 können durch einen gemeinsamen Prozessor gegebenenfalls mit einer geeigneten Programmierung realisiert werden.

Das Mundstück 1 weist eine Vielzahl von oralen Sensoren 16, 18, 20, 22, 24, 26, 28, 30, 32, 34 auf, die an verschiedenen Positionen des Mundstücks 1 an dessen Oberfläche und bevorzugt auch im Inneren des Mundstücks 1 angeordnet sind, so dass die oralen Sensoren 16, 18, 20, 22, 24, 26, 28, 30, 32, 34 sich im Mundraum der Person an unterschiedlichen Stellen befinden, wenn das Mundstück 1 ordnungsgemäß eingesetzt ist. Ordnungsgemäß eingesetzt bedeutet vorliegend, dass das Mundstück 1 mittels der Diagnoseschiene / Aktionsschiene 10 passend auf die okklusalen Seiten der zu den Zahnaufnahmevertiefungen 11 passenden Zähne der Person aufgesteckt ist. Beispielsweise können ein Elektromyographie-Sensor 16 (EMG) zur Messung von Muskelaktivitäten im Mundraum vorgesehen sein und eine Vielzahl von Oral-Neuro-Sensoren 20 zur Messung von neuronalen Aktivitäten einzelner Zahnnerven oder anderer Nerven im Mundraum. Hierzu ist vorzugsweise in oder an jeder der Zahnaufnahmevertiefungen 11 im Bereich des Zahnhalses oder der Gingiva-Linie (am Marginalsaum) ein separater Oral-Neuro-Sensor 20 angeordnet. Auch kann ein Zungensensor 22 zur Bestimmung der Lage der Zunge im Mundraum der Person und/oder zur Messung von neuronalen Aktivitäten von Nerven in der Zunge vorgesehen sein. Des Weiteren können im Bereich der Aufnahmen für die okklusalen Oberflächen von Molaren (Backenzähnen) Bisskraftsensoren 26 zur Bestimmung der Bisskraft vorgesehen sein. Ferner kann bevorzugt ein nicht invasiver Blutdrucksensor 28 vorgesehen sein, mit dem der Blutdruck der Person bestimmbar ist. Auch kann ein Lautstärkesensor 30 zur Bestimmung der Lautstärke von Schnarchen oder Knirschen der Person vorgesehen sein. Des Weiteren kann ein Temperatursensor 32 zur Bestimmung der Körpertemperatur der Person am Mundstück 1 angeordnet sein. Ferner kann ein Feuchtigkeitssensor 34 zur Bestimmung der Feuchtigkeit und des Speichelflusses im Mundraum der Person am Mundstück 1 vorgesehen sein.

Die oralen Sensoren 16, 18, 20, 22, 24, 26, 28, 30, 32, 34 können lösbar in Ausnehmungen 35 im Mundstück 1 befestigt sein und auf diese Weise austauschbar sein. Hierzu weist das Mundstück 1 eine Vielzahl von Ausnehmungen 35 auf, wobei in einige oder auch in alle der Ausnehmungen 35 jeweils einer der oralen Sensoren 16, 18, 20, 22, 24, 26, 28, 30, 32, 34 eingesetzt ist. Bevorzugt können die Ausnehmungen 35 hierzu eine zu einer äußeren Oberfläche von Halterungen (nicht gezeigt) der oralen Sensoren 16, 18, 20, 22, 24, 26, 28, 30, 32, 34 passende innere Oberflächen aufweisen, so dass die oralen Sensoren 16, 18, 20, 22, 24, 26, 28, 30, 32, 34 flächenbündig in die Ausnehmungen 35 einsetzbar sind. Besonders bevorzugt weisen die Ausnehmungen 35 die gleichen inneren Oberflächen auf und die oralen Sensoren 16, 18, 20, 22, 24, 26, 28, 30, 32, 34 oder zumindest diejenigen, die austauschbar sein sollen, weisen ebenfalls die gleichen äußeren Oberfläche an den Halterungen auf. Insbesondere wird eine konische Form der inneren Oberflächen der Ausnehmungen 35 vorgeschlagen und eine dazu passende konische äußere Form der Halterungen der oralen Sensoren 16, 18, 20, 22, 24, 26, 28, 30, 32, 34. Durch einen unterschiedlichen maximalen äußeren Durchmesser der konischen Form können die austauschbaren oralen Sensoren 16, 18, 20, 22, 24, 26, 28, 30, 32, 34 dann in unterschiedlicher Einstecktiefe in den Ausnehmungen 35 positioniert werden und damit an verschiedenen Orten im Mundraum positioniert werden. Die Befestigung der Halterungen der oralen Sensoren 16, 18, 20, 22, 24, 26, 28, 30, 32, 34 in den Ausnehmungen 35 im Mundstück 1 kann beispielsweise mit Hilfe eines Klebstoffs oder eines Haftvermittlers erfolgen oder auch durch Presspassung oder Klemmverbindung oder magnetisch.

Zusätzlich weist das Mundstück 1 erfindungsgemäß bevorzugt eine Vielzahl von Aktoren 42, 44, 46 in Form von Elektroden, Elektromagneten und/oder Medikamentenabgabe-Einrichtungen auf. Die Aktoren 42, 44, 46 können von der Steuerung 15 aktiviert werden. Die oralen Sensoren 16, 18, 20, 22, 24, 26, 28, 30, 32, 34 und die Aktoren 42, 44, 46 können bei Bedarf mit Strom aus dem elektrische Energiespeicher 12 versorgt werden. Mit Hilfe der Aktoren 42, 44, 46 können das Schlafverhalten und/oder der Gesundheitszustand der Person beeinflusst werden. Gleichzeitig kann mit dem oralen Sensoren 16, 18, 20, 22, 24, 26, 28, 30, 32, 34 die Wirkung der Aktoren 42, 44, 46 auf den Schlaf der Person gemessen und mit Hilfe der Auswertungseinrichtung 14 quantifiziert werden.

Die Aktoren 42, 44, 46 und einige der oralen Sensoren 22, 32 können auf Fortsätzen 36, 38, 40 angeordnet sein, die sich von der Diagnoseschiene / Aktionsschiene 10 ausgehend in den Mundraum der Person erstrecken, um bestimmte Punkte im Mundraum der Person zu erreichen, die mit den Sensoren 22, 32 überwacht werden sollen und/oder die mit den Aktoren 42, 44, 46 aktiviert werden sollen. Beispielweise kann der Zungenmuskel mit Hilfen von Elektroden 44 an einem hinteren lingualen Fortsatz 36 und/oder an einem vorderen lingualen Fortsatz 38 aktiviert werden. Die Position der Zunge kann mit Hilfe des Zungensensors 22 an vorderen lingualen Fortsätzen 38 bestimmt werden. Bevorzugt sind die lingualen Fortsätze 38 unter der Zunge der Person angeordnet, wenn das Mundstück 1 ordnungsgemäß in den Mundraum eingesetzt ist. Mit Hilfe eines proximalen Fortsatzes 40 kann ein Aktor 42 im Bereich des Kiefergelenks angeordnet werden. Zudem können am proximalen Fortsatz 40 auch orale Sensoren (nicht gezeigt) angeordnet sein, die eine Messung von neuronalen Aktivitäten oder anderen oralen Parametern an dieser Stelle erlauben. Es können auch zusätzlich externe Aktoren wie Vibrationseinrichtungen oder Leuchtdioden vorgesehen sein, mit denen das Schlafverhalten der Person von extern beeinflusst wird. Es ist auch möglich, Schluckimpulse mit Hilfe von oralen Aktoren anzuregen beziehungsweise auszulösen.

Auch die Aktoren 42, 44, 46 können gegebenenfalls in den Ausnehmungen 35 des Mundstücks 1 austauschbar angeordnet werden. Bevorzugt haben die Aktoren 42, 44, 46 hierzu eine Halterung mit einer äußeren Oberfläche, die zur inneren Oberfläche der Ausnehmungen 35 passt. Besonders bevorzugt können die äußeren Oberflächen der Halterungen der Aktoren 42, 44, 46 und die äußeren Oberflächen der Halterungen der austauschbaren oralen Sensoren 16, 18, 20, 22, 24, 26, 28, 30, 32, 34 die gleiche oder eine gleichartige Form aufweisen, so dass diese in den gleichen Ausnehmungen 35 befestigt werden können.

In den meisten Fällen ist die beidseitige Anordnung von oralen Sensoren 16, 18, 20, 22, 24, 26, 28, 30, 32, 34, Fortsätzen 36, 38, 40 und Aktoren 42, 44, 46 vorteilhaft, weil hierdurch Mittelwerte gebildet werden können oder bei einem schlechten oder einem fehlenden Kontakt auf einen anderen oralen Sensor 16, 18, 20, 22, 24, 26, 28, 30, 32, 34 oder Aktor 42, 44, 46 zurückgegriffen werden kann.

Als orale Sensoren 16, 18, 20, 22, 24, 26, 28, 30, 32, 34 können Lautstärkesensoren zur Messung der Lautstärke von Schnarchen und/oder Knirschen, Bisskraftsensoren 26 zur Messung der von den Kiefermuskels der Person ausgeübten Kraft auf die Zähne, Bewegungssensoren zur Messung der Bewegung des Unterkiefers gegen die Oberkiefer, Temperatursensoren 32 zur Messung der Körpertemperatur im Mundraum, Feuchtigkeitssensoren zur Messung der Feuchtigkeit beziehungsweise des Speichelflusses im Mundraum, Puls-Sauerstoffsensoren zur Messung der Pulsfrequenz und der Sauerstoffsättigung im Blut der Person, Vibrations- und Luftstromsensoren zur Messungen von Vibrationen bestimmter Frequenzen und zur Messung der Mundatmung, pH-Sensoren zur Messung des Säuregehalts im Mundraum, Lagesensoren zur Bestimmung der Lage und von Lageänderungen des Kopfs der Person, Ultraschallsensoren zur Bestimmung der Position und Ausdehnung der Zunge im Mundraum, Druckplattensensoren zur Bestimmung der Lage und der Versteifung oder der Spannung der Zunge, Pulslaufzeitsensoren zur Messung der Herzfrequenz, Sensoren zur Bestimmung der Färbung der Zunge und/oder der Mundschleimhäute, Atemalkoholsensoren zur Bestimmung des Atemalkohols, Blutalkoholsensoren, Elektromyographie-Sensoren 16 (EMG) zur Bestimmung der Aktivität von Muskeln im Mundraum der Person, Elektrokardiographie-Sensoren (EKG) zur Bestimmung der Herzaktivität, Elektroenzephalographie-Sensoren (EEG) zur Bestimmung von Hirnaktivitäten der Person, Milchsäure-Sensoren zur Bestimmung der Milchsäurekonzentration, nichtinvasive systolische/diastolische Blutdrucksensoren zur Bestimmung des Blutdrucks der Person, Kameras zur Bestimmung der Färbung der Zunge und/oder der Mundschleimhaut und/oder zur Bestimmung der Lage zur Zunge im Mundraum, Blutzuckersensoren zur Bestimmung des Blutzuckergehalts der Person sowie Oral-Neuro-Sensoren 20 zur Messung der neuronalen Aktivität von Zahnnerven einzelner Zähne oder anderer Nerven im Mundraum der Person verwendet werden.

Die Auswertungseinrichtung 14 des Mundstücks 1 ist bevorzugt zur Auswertung und/oder Analyse von oralen Messdaten der oralen Sensoren 16, 18, 20, 22, 24, 26, 28, 30, 32, 34 geeignet. Die Rohdaten oder die ausgewerteten oralen Messdaten können bevorzugt drahtlos über die Sender- / Empfängereinrichtung 4 des Mundstücks 1 an die Sender- / Empfängereinrichtung 6 des externen Außengeräts 2 übertragen werden.

Das externe Außengerät 2 kann ein Gehäuse 50 aufweisen, das das externe Außengerät 2 im Wesentlichen nach außen abschließt. Das externe Außengerät 2 kann ferner eine Steuerung 52 und eine Auswertungseinrichtung 54 aufweisen, die beide durch einen gemeinsamen Prozessor realisiert werden können.

Das externe Außengerät 2 weist einen oder mehrere externe Sensoren 56, 58, 60, 62, 64, 66 auf, die in oder an dem Gehäuse 50 angeordnet sein können. Zusätzlich weist das externe Überwachungsgerät 3 einen externen Sensor 68 in Form eines Schlafüberwachungssensors zum Bestimmen der Schlafphase der zweiten Person als externen Parameter auf. Mit diesen externen Sensoren 56, 58, 60, 62, 64, 66, 68 werden die physikalische Größen in der Umgebung der Person beziehungsweise in dem Raum, in dem die Person schläft, gemessen.

Als externe Sensoren 56, 58, 60, 62, 64, 66, 68 können Temperatursensoren 56 zur Messung der Umgebungstemperatur beziehungsweise der Raumtemperatur, Luftdrucksensoren 58 zur Messung des Luftdrucks der Umgebung oder in dem Raum, Luftfeuchtigkeitssensoren 60 zur Messung der Luftfeuchtigkeit in der Raumluft oder Umgebungsluft, Lautstärke-Sensoren oder Mikrofone 62 zur Messung von Schallpegeln und Geräuschen in der Umgebung, Helligkeitssensoren 64 zur Messung der Lichtstärke in der Umgebung oder in dem Raum, Chronometer 66, Chronometer zur Uhrzeit-Bestimmung, Chronometer zur Kalendertag-Bestimmung, Chronometer zur Mondstands-Bestimmung, Bewegungssensoren zur Messungen von Bewegungen in der Umgebung, Radar zur Bestimmung der Position der Person und Messung des Hebens und Senkens des Brustkorbs der Person, Sensoren zur Messung der Sauerstoffkonzentration in der Umgebungsluft, Sensoren zur Messung der Staubkonzentration in der Umgebungsluft, Sensoren zur Messung der CO₂-Konzentration in der Umgebungsluft, Timer, wie zum Beispiel Wecker und Kameras zur Bestimmung der Position und Lage der Person verwendet werden.

Die Auswertungseinrichtung 54 des externen Außengeräts 2 ist zur Auswertung und/oder Analyse von externen Messdaten der externen Sensoren 56, 58, 60, 62, 64, 66, 68 geeignet. Bevorzugt ist die Auswertungseinrichtung 54 des externen Außengeräts 2 auch zur Auswertung und Analyse der oralen Messdaten der oralen Sensoren 16, 18, 20, 22, 24, 26, 28, 30, 32, 34 geeignet und/oder zur Analyse von Auswertungen der oralen Messdaten geeignet, die bereits von der Auswertungseinrichtung 14 des Mundstücks 1 vorgenommen wurden. Zudem können vorzugsweise die oralen Aktoren 42, 44, 46 und gegebenenfalls vorhandene externe Aktoren (nicht gezeigt) von der Steuerung 52 des externen Außengeräts 2 aktiviert werden. Die Aktivierung erfolgt erfindungsgemäß auf Basis der Analyse und Korrelation der oralen und externen Messdaten.

Bevorzugt erfolgt bei der Auswertung und Analyse der oralen Messdaten der oralen Sensoren 16, 18, 20, 22, 24, 26, 28, 30, 32, 34 und der externen Messdaten der externen Sensoren 56, 58, 60, 62, 64, 66, 68 eine Auswertung des zeitlichen Verlaufs der oralen und externen Messdaten.

Das zumindest eine externe Überwachungsgerät 3 kann auch zusätzlich zu dem zumindest einen externen Sensor 68 einen weiteren Sensor in Form einer Schlafzyklus-Messeinrichtung zur Bestimmung des Schlafzyklus beziehungsweise einer Schlafphase zumindest einer zweiten Person im gleichen Raum oder in der Umgebung der (ersten) Person aufweisen. Die externen Messdaten der Schlafzyklus-Messeinrichtung beziehungsweise des externen Sensors 68 werden mittels der Sender- / Empfängereinrichtung 8 an das externe Außengerät 2 übertragen und dort von der Auswertungseinrichtung 54 ausgewertet und analysiert.

Das externe Außengerät 2 kann eine Anzeigeeinrichtung 70 zur graphischen Darstellung der Analysen aufweisen. Das Ergebnis der Analysen kann aber auch in Form von Daten übertragen werden, beispielsweise auf ein Smartphone (nicht gezeigt) oder einen Computer (nicht gezeigt). Dort können die Ergebnisse der Analysen der Person, einem Arzt oder einem Schlaflabor zur weiteren Verwendung zur Verfügung gestellt werden. Diese können dann mittels einer Eingabeeinrichtung (nicht gezeigt) Eingriffsgrenzwerte oder bestimmte Korrelationen anpassen und beeinflussen.

Die Auswertungseinrichtung 14, 54 ist dazu eingerichtet beziehungsweise dazu programmiert, aus den ausgewerteten oralen Messdaten den Schlafzustand und Informationen zum Schlafverhalten und/oder zum Gesundheitszustand der Person abzuleiten und diese mit den externen Messdaten und/oder externen Parametern in Korrelation zu setzen und auf diese Weise Einflussfaktoren der Umgebung beziehungsweise der externen Parameter auf den Gesundheitszustand und/oder das Schlafverhalten festzustellen und zur Analyse und zur Beeinflussung des Gesundheitszustands und/oder des Schlafverhaltens mit den oralen Aktoren 42, 44, 46 und/oder den externen Aktoren zu nutzen. Gleichzeitig kann die Wirkung des Einsatzes der oralen Aktoren 42, 44, 46 und der externen Aktoren auf den Gesundheitszustand und/oder das Schlafverhalten mit Hilfe der oralen Sensoren 16, 18, 20, 22, 24, 26, 28, 30, 32, 34 gemessen werden. All diese Daten und Analysen können zur Individualisierung von Analysen späterer Schlafvorgänge der Person oder späterer Messungen gespeichert werden. Es wird im Rahmen der vorliegenden Erfindung vorgeschlagen, zur Analyse und Auswertung der umfangreichen Messdaten auf eine künstliche Intelligenz zurückzugreifen, die Muster in den Daten erkennt, und bestimmten Gesundheitszuständen und/oder Schlafzuständen zuordnet. Auf diese Weise kann ein selbstoptimierendes und selbstlernendes Gesundheitsüberwachungssystem bereitgestellt werden, das mit der Zeit immer besser auf die Gesundheitszustände und/oder die Schlafbedürfnisse der Person abgestimmt ist. Eine externe Überwachung kann auch mittels einer geeigneten Smartwatch oder einer Matte mit darin verteilten Drucksensoren zur Messung der Lage der ersten Person oder der ersten Person und der zumindest einen zweiten Person im Bett erfolgen. Ferner kann auch ein Vergleich der Messdaten der ersten Person mit den Messdaten der zumindest einen zweiten Person erfolgen, um daraus Rückschlüsse zu ziehen.

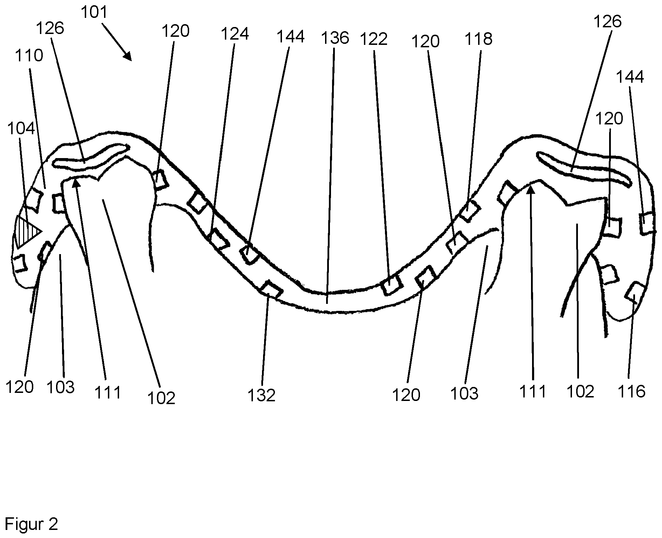

Figur 2 zeigt eine schematische Querschnittansicht eines alternativen Mundstücks 101 eines zweiten erfindungsgemäßen Gesundheitsüberwachungssystems, wobei das Mundstück 101 ordnungsgemäß im Mundraum einer Person eingesetzt ist. Hierzu kann das Mundstück 101 Zahnaufnahmevertiefungen 111 aufweisen, die auf Zähne 102 der Person aufgesteckt werden. Zusätzlich kann das Mundstück 101 flächenbündig auf der Gingiva 103 und Teilen der Mukosa des Mundraums der Person aufliegen und bevorzugt dort auch anhaften.

Das zweite erfindungsgemäße Gesundheitsüberwachungssystem weist analog der ersten Ausführung nach Figur 1 ein externes Außengerät (in Figur 2 nicht gezeigt) mit mehreren externen Sensoren und einer Auswertungseinrichtung auf und kann zusätzlich auch ein externes Überwachungsgerät aufweisen. Das externe Außengerät kann analog dem externen Außengerät 2 nach Figur 1 aufgebaut sein, wobei die externen Sensoren auch variieren können.

Das Mundstück 101 ist zum Einsetzen in den Mundraum der Person geeignet und vorgesehen. Insbesondere ist das Mundstück 101 zum Befestigen an den Zähnen 102 einer Zahnleiste eines Oberkiefers der Person geeignet und vorgesehen. Das Mundstück 101 weist eine Gaumenplatte 136 auf, die an den Gaumen der Person angelegt ist und die bevorzugt vor dem Gaumensegel (in Figur 2 nicht zu sehen) endet. Das externe Außengerät (nicht gezeigt) ist dafür vorgesehen, in der Umgebung der Person (im gleichen Raum) angeordnet zu werden, in dem die Person schläft oder sich aufhält und während die Person schläft oder wacht. Das externe Überwachungsgerät (nicht gezeigt) dient gegebenenfalls der Überwachung des Schlafzustands einer zweiten Person, die sich in dem gleichen Raum (in der gleichen Umgebung) aufhält wie die erste Person, oder zur Überwachung des Schlafzustands mehrerer zweiter Personen, die sich in dem gleichen Raum (in der gleichen Umgebung) aufhalten beziehungsweise schlafen wie die erste Person.

Das Mundstück 101 und das externe Außengerät weisen bevorzugt alle jeweils eine Sender-/ Empfängereinrichtung 104 auf, über die das Mundstück 101 zum Austausch von Daten mit dem externen Außengerät verbunden ist beziehungsweise bei Bedarf verbunden werden kann. Bevorzugt wird hierzu eine Drahtlos-Verbindung, wie eine Funkverbindung, Bluetooth oder WLAN genutzt, um einen Datenaustausch zwischen dem Mundstück 101 und dem externen Außengerät zu ermöglichen. Die Datenverbindung über die Sender- / Empfängereinrichtung 104 muss nicht immer beziehungsweise nicht kontinuierlich aktiv sein, sondern kann temporär zum Austausch von Daten genutzt werden.

Das Mundstück 101 kann bevorzugt eine anatomisch individuell angepasste Diagnoseschiene / Aktionsschiene 110 mit Zahnaufnahmevertiefungen 111 aufweisen. Bevorzugt ist die Diagnoseschiene / Aktionsschiene 110 eine Aufbissschiene. Theoretisch kann das Mundstück 101 aber auch ohne Diagnoseschiene / Aktionsschiene 110 realisiert werden und in seiner äußeren Form dennoch an die Oberfläche des Mundraums der Person angepasst sein. Die okklusalen Seiten beziehungsweise die okklusalen Oberflächen der Zähne 102 der Person werden bei der Ausführung mit Diagnoseschiene / Aktionsschiene 110 bevorzugt formschlüssig und flächenbündig in den Zahnaufnahmevertiefungen 111 aufgenommen, so wie das in Figur 2 zu erkennen ist. Auf diese Weise kann die Diagnoseschiene / Aktionsschiene 110 und damit das gesamte Mundstück 101 von den Zähnen 102 im Mundraum der Person gehalten werden. Bevorzugt liegt das Mundstück 101 auch noch zusätzlich an anderen Bereichen des Mundraums der Person (auch auf der Mukosa) an, wenn das Mundstück 101, wie in Figur 2 gezeigt, ordnungsgemäß im Mundraum der Person eingesetzt ist. Die Diagnoseschiene / Aktionsschiene 110 kann gleichzeitig die Zähne 102 der Person während des Schlafs vor einem Abrieb durch Bruxismus schützen. Bevorzugt wird die Diagnoseschiene / Aktionsschiene 110 aus einem Kunststoff, insbesondere auch einem Polyamid-12-Material oder aus Poly-Laurylactam, basierend auf einem individuellen 3D-Modell eines Teils des Mundraums der Person 3D-gedruckt, um eine besonders stabile und abriebfeste Diagnoseschiene / Aktionsschiene 110 zur Verfügung zu stellen. Neben dem 3D-Druck kann die Diagnoseschiene / Aktionsschiene 110 auch 3D-gefräst werden oder durch Spritzgussverfahren oder mit Streuverfahren hergestellt werden. Die Diagnoseschiene / Aktionsschiene 110 oder zumindest alle außenliegenden Teile des Mundstücks 101 werden aus biokompatiblen Materialien hergestellt.

Eingebettet in das Material des Mundstücks 101 oder der Diagnoseschiene / Aktionsschiene 110 kann ein elektrischer Energiespeicher (nicht gezeigt) in Form eines Akkumulators angeordnet sein. Der elektrische Energiespeicher ist bevorzugt drahtlos aufladbar (beispielsweise mit einem Ladegerät analog einer Handyschale) und dient der Spannungsversorgung der Sender- / Empfängereinrichtung 104 des Mundstücks 101 sowie optional einer Steuerung (nicht gezeigt) in Form eines Prozessors. Die Steuerung kann ebenfalls in ein Kunststoffmaterial des Mundstücks 101 oder der Diagnoseschiene / Aktionsschiene 110 eingebettet sein.

Das Mundstück 101 weist eine Vielzahl von oralen Sensoren 116, 118, 120, 122, 124, 126, 132 auf, die an verschiedenen Positionen des Mundstücks 101 an dessen Oberfläche angeordnet sind, so dass die oralen Sensoren 116, 118, 120, 122, 124, 126, 132 sich im Mundraum der Person an unterschiedlichen Stellen befinden, wenn das Mundstück 101 ordnungsgemäß eingesetzt ist. Ordnungsgemäß eingesetzt bedeutet vorliegend, dass das Mundstück 101 mittels der Diagnoseschiene / Aktionsschiene 110 passend auf die okklusalen Seiten der zu den Zahnaufnahmevertiefungen 111 passenden Zähne 102 der Person aufgesteckt ist. Beispielsweise können ein Elektromyographie-Sensor 116 (EMG) zur Messung von Muskelaktivitäten im Mundraum vorgesehen sein und eine Vielzahl von Oral-Neuro-Sensoren 120 zur Messung von neuronalen Aktivitäten einzelner Zahnnerven oder anderer Nerven im Mundraum. Hierzu ist vorzugsweise in oder an mehreren der Zahnaufnahmevertiefungen 111 im Bereich des Zahnhalses oder der Gingiva-Linie (am Marginalsaum) ein separater Oral-Neuro-Sensor 120 angeordnet. Auch kann ein Zungensensor 122 zur Bestimmung der Lage der Zunge im Mundraum der Person und/oder zur Messung von neuronalen Aktivitäten von Nerven in der Zunge vorgesehen sein. Des Weiteren können im Bereich der Aufnahmen für die okklusalen Oberflächen von Molaren (Backenzähnen) Bisskraftsensoren 126 zur Bestimmung der Bisskraft vorgesehen sein.

Ferner kann bevorzugt ein nicht invasiver Blutdrucksensor (nicht gezeigt) vorgesehen sein, mit dem der Blutdruck der Person bestimmbar ist. Des Weiteren kann ein Temperatursensor 132 zur Bestimmung der Körpertemperatur der Person am Mundstück 101 angeordnet sein. Ferner kann ein Feuchtigkeitssensor (nicht gezeigt) zur Bestimmung der Feuchtigkeit und des Speichelflusses im Mundraum der Person am Mundstück 101 vorgesehen sein.

Die oralen Sensoren 116, 118, 120, 122, 124, 126, 132 können lösbar in Ausnehmungen im Mundstück 101 befestigt sein und auf diese Weise austauschbar sein. Hierzu weist das Mundstück 101 eine Vielzahl von Ausnehmungen auf, wobei in einige oder auch in alle der Ausnehmungen jeweils einer der oralen Sensoren 116, 118, 120, 122, 124, 126, 132 eingesetzt ist beziehungsweise werden kann. Bevorzugt können die Ausnehmungen hierzu eine zu einer äußeren Oberfläche von Halterungen (nicht gezeigt) der oralen Sensoren 116, 118, 120, 122, 124, 126, 132 passende innere Oberflächen aufweisen, so dass die oralen Sensoren 116, 118, 120, 122, 124, 126, 132 flächenbündig in die Ausnehmungen einsetzbar sind. Besonders bevorzugt weisen die Ausnehmungen die gleichen inneren Oberflächen auf und die oralen Sensoren 116, 118, 120, 122, 124, 126, 132 oder zumindest diejenigen, die austauschbar sein sollen, weisen ebenfalls die gleichen äußeren Oberfläche an den Halterungen auf. Insbesondere wird eine konische Form der inneren Oberflächen der Ausnehmungen vorgeschlagen und eine dazu passende konische äußere Form der Halterungen der oralen Sensoren 116, 118, 120, 122, 124, 126, 132. Durch einen unterschiedlichen maximalen äußeren Durchmesser der konischen Form können die austauschbaren oralen Sensoren 116, 118, 120, 122, 124, 126, 132 dann in unterschiedlicher Einstecktiefe in den Ausnehmungen positioniert werden und damit an verschiedenen Orten im Mundraum positioniert werden. Die Befestigung der Halterungen der oralen Sensoren 116, 118, 120, 122, 124, 126, 132 in den Ausnehmungen im Mundstück 101 kann beispielsweise mit Hilfe eines Klebstoffs oder eines Haftvermittlers erfolgen oder auch durch Presspassung oder Klemmverbindung oder magnetisch.

Zusätzlich weist das Mundstück 101 erfindungsgemäß bevorzugt mehrere Aktoren 144 in Form von Elektroden, Elektromagneten und/oder Medikamentenabgabe-Einrichtungen auf. Die Aktoren 144 können von der Steuerung im Mundstück 101 oder im externen Außengerät aktiviert werden. Die oralen Sensoren 116, 118, 120, 122, 124, 126, 132 und die Aktoren 144 können bei Bedarf mit Strom aus dem elektrische Energiespeicher versorgt werden. Mit Hilfe der Aktoren 144 können das Schlafverhalten und/oder der Gesundheitszustand der Person beeinflusst werden. Gleichzeitig kann mit dem oralen Sensoren 116, 118, 120, 122, 124, 126, 132 die Wirkung der Aktoren 144 auf den Schlaf der Person gemessen und mit Hilfe der Auswertungseinrichtung des externen Außengeräts quantifiziert werden.

Einige der Aktoren 144 und einige der oralen Sensoren 120, 122, 124, 132 können auf der Gaumenplatte 136 angeordnet sein, die sich von der Diagnoseschiene / Aktionsschiene 110 ausgehend in den Mundraum der Person erstreckt, um bestimmte Punkte im Mundraum der Person zu erreichen, die mit den Sensoren 120, 122, 124, 132 überwacht werden sollen und/oder die mit den Aktoren 144 aktiviert werden sollen. Die Position der Zunge kann mit Hilfe des Zungensensors 122 an der Gaumenplatte 136 bestimmt werden. Bevorzugt ist die Gaumenplatte 136 oberhalb der Zunge der Person angeordnet, wenn das Mundstück 101 ordnungsgemäß in den Mundraum eingesetzt ist. Es können auch zusätzlich externe Aktoren wie Vibrationseinrichtungen oder Leuchtdioden vorgesehen sein, mit denen das Schlafverhalten der Person von extern beeinflusst wird. Es ist auch möglich, Schluckimpulse mit Hilfe von oralen Aktoren anzuregen beziehungsweise auszulösen.

Auch die Aktoren 144 können gegebenenfalls in den Ausnehmungen des Mundstücks 101 austauschbar angeordnet werden. Bevorzugt haben die Aktoren 144 hierzu eine Halterung mit einer äußeren Oberfläche, die zur inneren Oberfläche der Ausnehmungen passt. Besonders bevorzugt können die äußeren Oberflächen der Halterungen der Aktoren 144 und die äußeren Oberflächen der Halterungen der austauschbaren oralen Sensoren 116, 118, 120, 122, 124, 126, 132 die gleiche oder eine gleichartige Form aufweisen, so dass diese in den gleichen Ausnehmungen befestigt werden können.

In den meisten Fällen ist die beidseitige Anordnung von oralen Sensoren 116, 118, 120, 122, 124, 126, 132 und Aktoren 144 vorteilhaft, weil hierdurch Mittelwerte gebildet werden können oder bei einem schlechten oder einem fehlenden Kontakt auf einen anderen oralen Sensor 116, 118, 120, 122, 124, 126, 132 oder Aktor 144 zurückgegriffen werden kann.

Als orale Sensoren 116, 118, 120, 122, 124, 126, 132 können Lautstärkesensoren zur Messung der Lautstärke von Schnarchen und/oder Knirschen, Bisskraftsensoren 126 zur Messung der von den Kiefermuskels der Person ausgeübten Kraft auf die Zähne, Bewegungssensoren zur Messung der Bewegung des Unterkiefers gegen die Oberkiefer, Temperatursensoren 132 zur Messung der Körpertemperatur im Mundraum, Feuchtigkeitssensoren zur Messung der Feuchtigkeit beziehungsweise des Speichelflusses im Mundraum, Puls-Sauerstoffsensoren zur Messung der Pulsfrequenz und der Sauerstoffsättigung im Blut der Person, Vibrations- und Luftstromsensoren zur Messungen von Vibrationen bestimmter Frequenzen und zur Messung der Mundatmung, pH-Sensoren zur Messung des Säuregehalts im Mundraum, Lagesensoren zur Bestimmung der Lage und von Lageänderungen des Kopfs der Person, Ultraschallsensoren zur Bestimmung der Position und Ausdehnung der Zunge im Mundraum, Druckplattensensoren zur Bestimmung der Lage und der Versteifung oder der Spannung der Zunge, Pulslaufzeitsensoren zur Messung der Herzfrequenz, Sensoren zur Bestimmung der Färbung der Zunge und/oder der Mundschleimhäute, Atemalkoholsensoren zur Bestimmung des Atemalkohols, Blutalkoholsensoren, Elektromyographie-Sensoren 116 (EMG) zur Bestimmung der Aktivität von Muskeln im Mundraum der Person, Elektrokardiographie-Sensoren (EKG) zur Bestimmung der Herzaktivität, Elektroenzephalographie-Sensoren (EEG) zur Bestimmung von Hirnaktivitäten der Person, Milchsäure-Sensoren zur Bestimmung der Milchsäurekonzentration, nichtinvasive systolische/diastolische Blutdrucksensoren zur Bestimmung des Blutdrucks der Person, Kameras zur Bestimmung der Färbung der Zunge und/oder der Mundschleimhaut und/oder zur Bestimmung der Lage zur Zunge im Mundraum, Blutzuckersensoren zur Bestimmung des Blutzuckergehalts der Person sowie Oral-Neuro-Sensoren 120 zur Messung der neuronalen Aktivität von Zahnnerven einzelner Zähne oder anderer Nerven im Mundraum der Person verwendet werden.

Die Auswertungseinrichtung des externen Außengeräts ist zur Auswertung und/oder Analyse von oralen Messdaten der oralen Sensoren 116, 118, 120, 122, 124, 126, 132 geeignet. Die Rohdaten beziehungsweise die oralen Messdaten können bevorzugt drahtlos über die Sender-/ Empfängereinrichtung 104 des Mundstücks 101 an die Sender- / Empfängereinrichtung des externen Außengeräts übertragen werden.

Das externe Außengerät weist einen oder mehrere externe Sensoren auf. Zusätzlich kann das externe Überwachungsgerät einen externen Sensor in Form eines Schlafüberwachungssensors zum Bestimmen der Schlafphase der zweiten Person als externen Parameter aufweisen. Mit diesen externen Sensoren können die physikalische Größen in der Umgebung der Person beziehungsweise in dem Raum, in dem die Person schläft, gemessen werden.

Als externe Sensoren können Temperatursensoren zur Messung der Umgebungstemperatur beziehungsweise der Raumtemperatur, Luftfeuchtigkeitssensoren zur Messung der Luftfeuchtigkeit in der Raumluft oder Umgebungsluft, Lautstärke-Sensoren oder Mikrofone zur Messung von Schallpegeln und Geräuschen in der Umgebung, Helligkeitssensoren zur Messung der Lichtstärke in der Umgebung oder in dem Raum, Chronometer zur Bestimmung der Uhrzeit, des Kalendertags und/oder des Mondstands, Bewegungssensoren zur Messungen von Bewegungen in der Umgebung, Radar zur Bestimmung der Position der Person und Messung des Hebens und Senkens des Brustkorbs der Person, Sensoren zur Messung der Sauerstoffkonzentration in der Umgebungsluft und Sensoren zur Messung der CO₂-Konzentration in der Umgebungsluft verwendet werden.

Die Auswertungseinrichtung des externen Außengeräts ist zur Auswertung und/oder Analyse von externen Messdaten der externen Sensoren geeignet und ist auch zur Auswertung und Analyse der oralen Messdaten der oralen Sensoren 116, 118, 120, 122, 124, 126, 132 geeignet und/oder zur Analyse von Auswertungen der oralen Messdaten geeignet. Zudem können vorzugsweise die oralen Aktoren 144 und gegebenenfalls vorhandene externe Aktoren (nicht gezeigt) von der Steuerung des externen Außengeräts aktiviert werden. Die Aktivierung erfolgt erfindungsgemäß auf Basis der Analyse und Korrelation der oralen und externen Messdaten.

Bevorzugt erfolgt bei der Auswertung und Analyse der oralen Messdaten der oralen Sensoren 116, 118, 120, 122, 124, 126, 132 und der externen Messdaten der externen Sensoren eine Auswertung des zeitlichen Verlaufs der oralen und externen Messdaten.

Das externe Außengerät kann eine Anzeigeeinrichtung zur graphischen Darstellung der Analysen aufweisen. Das Ergebnis der Analysen kann aber auch in Form von Daten übertragen werden, beispielsweise auf ein Smartphone (nicht gezeigt) oder einen Computer (nicht gezeigt). Dort können die Ergebnisse der Analysen der Person, einem Arzt oder einem Schlaflabor zur weiteren Verwendung zur Verfügung gestellt werden. Diese können dann mittels einer Eingabeeinrichtung (nicht gezeigt) Eingriffsgrenzwerte oder bestimmte Korrelationen anpassen und beeinflussen.

Die Auswertungseinrichtung des externen Außengeräts ist dazu eingerichtet beziehungsweise dazu programmiert, aus den ausgewerteten oralen Messdaten den Schlafzustand und Informationen zum Schlafverhalten und/oder zum Gesundheitszustand der Person abzuleiten und diese mit den externen Messdaten und/oder externen Parametern in Korrelation zu setzen und auf diese Weise Einflussfaktoren der Umgebung beziehungsweise der externen Parameter auf den Gesundheitszustand und/oder das Schlafverhalten festzustellen und zur Analyse und zur Beeinflussung des Gesundheitszustands und/oder des Schlafverhaltens mit den oralen Aktoren 144 und/oder den externen Aktoren zu nutzen. Gleichzeitig kann die Wirkung des Einsatzes der oralen Aktoren 144 und der externen Aktoren auf den Gesundheitszustand und/oder das Schlafverhalten mit Hilfe der oralen Sensoren 116, 118, 120, 122, 124, 126, 132 gemessen werden. All diese Daten und Analysen können zur Individualisierung von Analysen späterer Schlafvorgänge der Person oder späterer Messungen gespeichert werden. Es wird im Rahmen der vorliegenden Erfindung vorgeschlagen, zur Analyse und Auswertung der umfangreichen Messdaten auf eine künstliche Intelligenz zurückzugreifen, die Muster in den Daten erkennt, und bestimmten Gesundheitszuständen und/oder Schlafzuständen zuordnet. Auf diese Weise kann ein selbstoptimierendes und selbstlernendes Gesundheitsüberwachungssystem bereitgestellt werden, das mit der Zeit immer besser auf die Gesundheitszustände und/oder die Schlafbedürfnisse der Person abgestimmt ist. Eine externe Überwachung kann auch mittels einer geeigneten Smartwatch oder einer Matte mit darin verteilten Drucksensoren zur Messung der Lage der ersten Person oder der ersten Person und der zumindest einen zweiten Person im Bett erfolgen. Ferner kann auch ein Vergleich der Messdaten der ersten Person mit den Messdaten der zumindest einen zweiten Person erfolgen, um daraus Rückschlüsse zu ziehen.

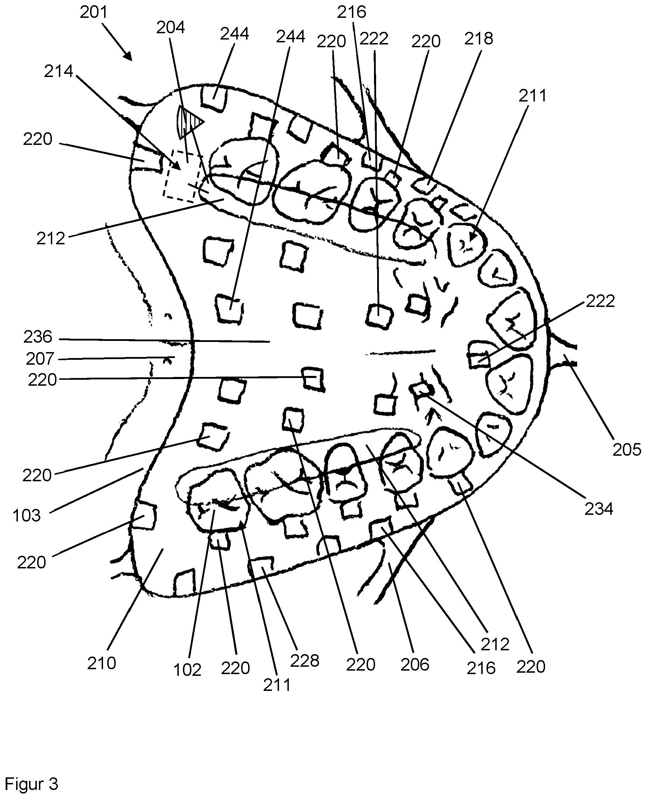

Figur 3 zeigt eine schematische, transparente Aufsicht auf ein weiteres alternatives Mundstück 201 eines dritten erfindungsgemäßen Gesundheitsüberwachungssystems, wobei das Mundstück 201 ordnungsgemäß im Mundraum einer Person eingesetzt ist. Hierzu kann das Mundstück 201 Zahnaufnahmevertiefungen 211 aufweisen, die auf Zähne 102 der Person aufgesteckt werden. Zusätzlich kann das Mundstück 201 flächenbündig auf der Gingiva 103 und Teilen der Mukosa des Mundraums der Person aufliegen und bevorzugt dort auch anhaften. Das Mundstück 201 kann geeignete randseitige Ausnehmungen aufweisen, um ein Lippenbändchen 205, die Wangenbändchen 206 und gegebenenfalls andere Bändchen aufzunehmen und diese im Mundraum nicht zu stören. Insbesondere kann ein Gaumensegel 207 der Person freigelassen werden, um keinen Würgereiz bei der Person zu provozieren.

Das dritte erfindungsgemäße Gesundheitsüberwachungssystem weist analog der ersten Ausführung nach Figur 1 ein externes Außengerät (in Figur 3 nicht gezeigt) mit mehreren externen Sensoren und einer externen Auswertungseinrichtung auf und kann zusätzlich auch ein externes Überwachungsgerät aufweisen. Das externe Außengerät kann analog dem externen Außengerät 2 nach Figur 1 aufgebaut sein, wobei die externen Sensoren teilweise variieren können.

Das Mundstück 201 ist zum Einsetzen in den Mundraum der Person geeignet und vorgesehen. Insbesondere ist das Mundstück 201 zum Befestigen an den Zähnen 102 einer Zahnleiste eines Oberkiefers der Person geeignet und vorgesehen. Das Mundstück 201 weist eine Gaumenplatte 236 auf, die an den Gaumen der Person angelegt ist und die bevorzugt vor dem Gaumensegel 207 endet. Das externe Außengerät (nicht gezeigt) ist dafür vorgesehen, in der Umgebung der Person (im gleichen Raum) angeordnet zu werden, in dem die Person schläft oder sich aufhält und während die Person schläft oder wacht. Das externe Überwachungsgerät (nicht gezeigt) dient gegebenenfalls der Überwachung des Schlafzustands einer zweiten Person, die sich in dem gleichen Raum (in der gleichen Umgebung) aufhält wie die erste Person, oder zur Überwachung des Schlafzustands mehrerer zweiter Personen, die sich in dem gleichen Raum (in der gleichen Umgebung) aufhalten beziehungsweise schlafen wie die erste Person.

Das Mundstück 201 und das externe Außengerät weisen bevorzugt alle jeweils eine Sender-/ Empfängereinrichtung 204 auf, über die das Mundstück 201 zum Austausch von Daten mit dem externen Außengerät verbunden ist beziehungsweise bei Bedarf verbunden werden kann. Bevorzugt wird hierzu eine Drahtlos-Verbindung, wie eine Funkverbindung, Bluetooth oder WLAN genutzt, um einen Datenaustausch zwischen dem Mundstück 201 und dem externen Außengerät zu ermöglichen. Die Datenverbindung über die Sender- / Empfängereinrichtung 204 muss nicht immer beziehungsweise nicht kontinuierlich aktiv sein, sondern kann temporär zum Austausch von Daten genutzt werden.

Das Mundstück 201 kann bevorzugt eine anatomisch individuell angepasste Diagnoseschiene / Aktionsschiene 210 mit Zahnaufnahmevertiefungen 211 aufweisen. Bevorzugt ist die Diagnoseschiene / Aktionsschiene 210 eine Aufbissschiene. Theoretisch kann das Mundstück 201 aber auch ohne Diagnoseschiene / Aktionsschiene 210 realisiert werden und in seiner äußeren Form dennoch an die Oberfläche des Mundraums der Person angepasst sein. Die okklusalen Seiten beziehungsweise die okklusalen Oberflächen der Zähne 102 der Person werden bei der Ausführung mit Diagnoseschiene / Aktionsschiene 210 bevorzugt formschlüssig und flächenbündig in den Zahnaufnahmevertiefungen 211 aufgenommen, so wie das in Figur 3 zu erkennen ist. Auf diese Weise kann die Diagnoseschiene / Aktionsschiene 210 und damit das gesamte Mundstück 201 von den Zähnen 102 im Mundraum der Person gehalten werden. Bevorzugt liegt das Mundstück 201 auch noch zusätzlich an anderen Bereichen des Mundraums der Person (auch auf der Mukosa) an, wenn das Mundstück 201, wie in Figur 3 gezeigt, ordnungsgemäß im Mundraum der Person eingesetzt ist. Die Diagnoseschiene / Aktionsschiene 210 kann gleichzeitig die Zähne 102 der Person während des Schlafs vor einem Abrieb durch Bruxismus schützen. Bevorzugt wird die Diagnoseschiene / Aktionsschiene 210 aus einem Kunststoff, insbesondere auch einem Polyamid-12-Material oder aus Poly-Laurylactam, basierend auf einem individuellen 3D-Modell eines Teils des Mundraums der Person 3D-gedruckt, um eine besonders stabile und abriebfeste Diagnoseschiene / Aktionsschiene 210 zur Verfügung zu stellen. Neben dem 3D-Druck kann die Diagnoseschiene / Aktionsschiene 210 auch 3D-gefräst werden oder durch Spritzgussverfahren oder mit Streuverfahren hergestellt werden. Die Diagnoseschiene / Aktionsschiene 210 oder zumindest alle außenliegenden Teile des Mundstücks 201 werden aus biokompatiblen Materialien hergestellt.

Eingebettet in das Material des Mundstücks 201 oder der Diagnoseschiene / Aktionsschiene 210 kann ein elektrischer Energiespeicher 212 in Form eines Akkumulators angeordnet sein. Der elektrische Energiespeicher ist in Figur 3 zweiteilig und auf beiden Seiten des Zahnbogens angeordnet. Der elektrische Energiespeicher 212 ist bevorzugt drahtlos aufladbar (beispielsweise mit einem Ladegerät analog einer Handyschale) und dient der Spannungsversorgung der Sender- / Empfängereinrichtung 204 des Mundstücks 201 sowie einer Auswertungseinrichtung 214 und einer Steuerung in Form eines Prozessors. Der elektrische Energiespeicher 212 kann mit Hilfe einer Vorrichtung zum Umwandeln einer körpereigenen Energiequelle in elektrische Energie (nicht gezeigt) mit elektrischer Energie aufgeladen werden. Es ist auch möglich, die Sender- / Empfängereinrichtung 204 des Mundstücks 201, die Auswertungseinrichtung 214 und die Steuerung in Form eines Prozessors mit elektrischer Spannung der Vorrichtung zum Umwandeln einer körpereigenen Energiequelle in elektrische Energie zu versorgen. Die körpereigene Energiequelle kann beispielsweise auf Muskelkraft, thermischen Prozessen oder chemischen Prozessen beruhen und mit Hilfe von mit Muskelkraft getriebenen Dynamos oder mit chemischen Reaktoren oder mit thermischen Stromerzeugern angezapft werden, um die notwendige Stromversorgung sicherzustellen oder zu verbessern. Die Auswertungseinrichtung 214 und die Steuerung können ebenfalls in ein Kunststoffmaterial des Mundstücks 201 oder der Diagnoseschiene / Aktionsschiene 210 eingebettet sein. Die Auswertungseinrichtung 214 und die Steuerung können durch einen gemeinsamen Prozessor gegebenenfalls mit einer geeignetem Programmierung realisiert werden.

Das Mundstück 201 weist eine Vielzahl von oralen Sensoren 216, 218, 220, 222, 228, 234 auf, die an verschiedenen Positionen des Mundstücks 201 an dessen Oberfläche angeordnet sind, so dass die oralen Sensoren 216, 218, 220, 222, 228, 234 sich im Mundraum der Person an unterschiedlichen Stellen befinden, wenn das Mundstück 201 ordnungsgemäß eingesetzt ist. Ordnungsgemäß eingesetzt bedeutet vorliegend, dass das Mundstück 201 mittels der Diagnoseschiene / Aktionsschiene 210 passend auf die okklusalen Seiten der zu den Zahnaufnahmevertiefungen 211 passenden Zähne 102 der Person aufgesteckt ist. Beispielsweise können ein Elektromyographie-Sensor 216 (EMG) zur Messung von Muskelaktivitäten im Mundraum vorgesehen sein und eine Vielzahl von Oral-Neuro-Sensoren 220 zur Messung von neuronalen Aktivitäten einzelner Zahnnerven oder anderer Nerven im Mundraum. Hierzu ist vorzugsweise in oder an mehreren der Zahnaufnahmevertiefungen 211 im Bereich des Zahnhalses oder der Gingiva-Linie (am Marginalsaum) ein separater Oral-Neuro-Sensor 220 angeordnet. Auch kann ein Zungensensor 222 zur Bestimmung der Lage der Zunge im Mundraum der Person und/oder zur Messung von neuronalen Aktivitäten von Nerven in der Zunge vorgesehen sein. Des Weiteren können im Bereich der Aufnahmen für die okklusalen Oberflächen von Molaren (Backenzähnen) Bisskraftsensoren zur Bestimmung der Bisskraft vorgesehen sein. Ferner kann bevorzugt ein nicht invasiver Blutdrucksensor 228 vorgesehen sein, mit dem der Blutdruck der Person bestimmbar ist. Des Weiteren kann ein Temperatursensor (nicht gezeigt) zur Bestimmung der Körpertemperatur der Person am Mundstück 201 angeordnet sein. Ferner kann ein Feuchtigkeitssensor 234 zur Bestimmung der Feuchtigkeit und des Speichelflusses im Mundraum der Person am Mundstück 201 vorgesehen sein.

Die oralen Sensoren 216, 218, 220, 222, 228, 234 können lösbar in Ausnehmungen im Mundstück 201 befestigt sein und auf diese Weise austauschbar sein. Hierzu weist das Mundstück 201 eine Vielzahl von Ausnehmungen auf, wobei in einige oder auch in alle der Ausnehmungen jeweils einer der oralen Sensoren 216, 218, 220, 222, 228, 234 eingesetzt ist beziehungsweise werden kann. Bevorzugt können die Ausnehmungen hierzu eine zu einer äußeren Oberfläche von Halterungen (nicht gezeigt) der oralen Sensoren 216, 218, 220, 222, 228, 234 passende innere Oberflächen aufweisen, so dass die oralen Sensoren 216, 218, 220, 222, 228, 234 flächenbündig in die Ausnehmungen einsetzbar sind. Besonders bevorzugt weisen die Ausnehmungen die gleichen inneren Oberflächen auf und die oralen Sensoren 216, 218, 220, 222, 228, 234 oder zumindest diejenigen, die austauschbar sein sollen, weisen ebenfalls die gleichen äußeren Oberfläche an den Halterungen auf. Insbesondere wird eine konische Form der inneren Oberflächen der Ausnehmungen vorgeschlagen und eine dazu passende konische äußere Form der Halterungen der oralen Sensoren 216, 218, 220, 222, 228, 234. Durch einen unterschiedlichen maximalen äußeren Durchmesser der konischen Form können die austauschbaren oralen Sensoren 216, 218, 220, 222, 228, 234 dann in unterschiedlicher Einstecktiefe in den Ausnehmungen positioniert werden und damit an verschiedenen Orten im Mundraum positioniert werden. Die Befestigung der Halterungen der oralen Sensoren 216, 218, 220, 222, 228, 234 in den Ausnehmungen im Mundstück 201 kann beispielsweise mit Hilfe eines Klebstoffs oder eines Haftvermittlers erfolgen oder auch durch Presspassung oder Klemmverbindung oder magnetisch.

Zusätzlich weist das Mundstück 201 erfindungsgemäß bevorzugt mehrere Aktoren 244 in Form von Elektroden, Elektromagneten und/oder Medikamentenabgabe-Einrichtungen auf. Die Aktoren 244 können von der Steuerung aktiviert werden. Die oralen Sensoren 216, 218, 220, 222, 228, 234 und die Aktoren 244 können bei Bedarf mit Strom aus dem elektrische Energiespeicher 212 versorgt werden. Mit Hilfe der Aktoren 244 können das Schlafverhalten und/oder der Gesundheitszustand der Person beeinflusst werden. Gleichzeitig kann mit dem oralen Sensoren 216, 218, 220, 222, 228, 234 die Wirkung der Aktoren 244 auf den Schlaf der Person gemessen und mit Hilfe der Auswertungseinrichtung 214 quantifiziert werden.

Einige der Aktoren 244 und einige der oralen Sensoren 220, 222 können auf der Gaumenplatte 236 angeordnet sein, die sich von der Diagnoseschiene / Aktionsschiene 210 ausgehend in den Mundraum der Person erstreckt, um bestimmte Punkte im Mundraum der Person zu erreichen, die mit den Sensoren 220, 222 überwacht werden sollen und/oder die mit den Aktoren 244 aktiviert werden sollen. Die Position der Zunge kann mit Hilfe des Zungensensors 222 an der Gaumenplatte 236 bestimmt werden. Bevorzugt ist die Gaumenplatte 236 oberhalb der Zunge der Person angeordnet, wenn das Mundstück 201 ordnungsgemäß in den Mundraum eingesetzt ist. Es können auch zusätzlich externe Aktoren wie Vibrationseinrichtungen oder Leuchtdioden vorgesehen sein, mit denen das Schlafverhalten der Person von extern beeinflusst wird. Es ist auch möglich, Schluckimpulse mit Hilfe von oralen Aktoren anzuregen beziehungsweise auszulösen.

Auch die Aktoren 244 können gegebenenfalls in den Ausnehmungen des Mundstücks 201 austauschbar angeordnet werden. Bevorzugt haben die Aktoren 244 hierzu eine Halterung mit einer äußeren Oberfläche, die zur inneren Oberfläche der Ausnehmungen passt. Besonders bevorzugt können die äußeren Oberflächen der Halterungen der Aktoren 244 und die äußeren Oberflächen der Halterungen der austauschbaren oralen Sensoren 216, 218, 220, 222, 228, 234 die gleiche oder eine gleichartige Form aufweisen, so dass diese in den gleichen Ausnehmungen befestigt werden können.

In den meisten Fällen ist die beidseitige Anordnung von oralen Sensoren 216, 218, 220, 222, 228, 234 und Aktoren 244 vorteilhaft, weil hierdurch Mittelwerte gebildet werden können oder bei einem schlechten oder einem fehlenden Kontakt auf einen anderen oralen Sensor 216, 218, 220, 222, 228, 234 oder Aktor 244 zurückgegriffen werden kann.

Als orale Sensoren 216, 218, 220, 222, 228, 234 können Lautstärkesensoren zur Messung der Lautstärke von Schnarchen und/oder Knirschen, Bisskraftsensoren zur Messung der von den Kiefermuskels der Person ausgeübten Kraft auf die Zähne, Bewegungssensoren zur Messung der Bewegung des Unterkiefers gegen die Oberkiefer, Temperatursensoren zur Messung der Körpertemperatur im Mundraum, Feuchtigkeitssensoren 234 zur Messung der Feuchtigkeit beziehungsweise des Speichelflusses im Mundraum, Puls-Sauerstoffsensoren zur Messung der Pulsfrequenz und der Sauerstoffsättigung im Blut der Person, Vibrations- und Luftstromsensoren zur Messungen von Vibrationen bestimmter Frequenzen und zur Messung der Mundatmung, pH-Sensoren zur Messung des Säuregehalts im Mundraum, Lagesensoren zur Bestimmung der Lage und von Lageänderungen des Kopfs der Person, Ultraschallsensoren zur Bestimmung der Position und Ausdehnung der Zunge im Mundraum, Druckplattensensoren zur Bestimmung der Lage und der Versteifung oder der Spannung der Zunge, Pulslaufzeitsensoren zur Messung der Herzfrequenz, Sensoren zur Bestimmung der Färbung der Zunge und/oder der Mundschleimhäute, Atemalkoholsensoren zur Bestimmung des Atemalkohols, Blutalkoholsensoren, Elektromyographie-Sensoren 216 (EMG) zur Bestimmung der Aktivität von Muskeln im Mundraum der Person, Elektrokardiographie-Sensoren (EKG) zur Bestimmung der Herzaktivität, Elektroenzephalographie-Sensoren (EEG) zur Bestimmung von Hirnaktivitäten der Person, Milchsäure-Sensoren zur Bestimmung der Milchsäurekonzentration, nichtinvasive systolische/diastolische Blutdrucksensoren 228 zur Bestimmung des Blutdrucks der Person, Kameras zur Bestimmung der Färbung der Zunge und/oder der Mundschleimhaut und/oder zur Bestimmung der Lage zur Zunge im Mundraum, Blutzuckersensoren zur Bestimmung des Blutzuckergehalts der Person sowie Oral-Neuro-Sensoren 220 zur Messung der neuronalen Aktivität von Zahnnerven einzelner Zähne oder anderer Nerven im Mundraum der Person verwendet werden.

Die Auswertungseinrichtung 214 des Mundstücks 201 ist bevorzugt zur Auswertung und/oder Analyse von oralen Messdaten der oralen Sensoren 216, 218, 220, 222, 228, 234 geeignet. Die Rohdaten oder die ausgewerteten oralen Messdaten können bevorzugt drahtlos über die Sender- / Empfängereinrichtung 204 des Mundstücks 201 an die Sender- / Empfängereinrichtung des externen Außengeräts übertragen werden.

Das externe Außengerät weist einen oder mehrere externe Sensoren auf. Zusätzlich kann das externe Überwachungsgerät einen externen Sensor in Form eines Schlafüberwachungssensors zum Bestimmen der Schlafphase der zweiten Person als externen Parameter aufweisen. Mit diesen externen Sensoren können die physikalische Größen in der Umgebung der Person beziehungsweise in dem Raum, in dem die Person schläft, gemessen werden.

Als externe Sensoren können Temperatursensoren zur Messung der Umgebungstemperatur beziehungsweise der Raumtemperatur, Luftfeuchtigkeitssensoren zur Messung der Luftfeuchtigkeit in der Raumluft oder Umgebungsluft, Lautstärke-Sensoren oder Mikrofone zur Messung von Schallpegeln und Geräuschen in der Umgebung, Helligkeitssensoren zur Messung der Lichtstärke in der Umgebung oder in dem Raum, Chronometer zur Bestimmung der Uhrzeit, des Kalendertags und/oder des Mondstands, Bewegungssensoren zur Messungen von Bewegungen in der Umgebung, Radar zur Bestimmung der Position der Person und Messung des Hebens und Senkens des Brustkorbs der Person, Sensoren zur Messung der Sauerstoffkonzentration in der Umgebungsluft und Sensoren zur Messung der CO₂-Konzentration in der Umgebungsluft verwendet werden.

Die Auswertungseinrichtung des externen Außengeräts ist zur Auswertung und/oder Analyse von externen Messdaten der externen Sensoren geeignet und bevorzugt ist die Auswertungseinrichtung des externen Außengeräts auch zur Auswertung und Analyse der oralen Messdaten der oralen Sensoren 216, 218, 220, 222, 228, 234 geeignet und/oder zur Analyse von Auswertungen der oralen Messdaten geeignet, die bereits von der Auswertungseinrichtung 214 im Mundstück 201 vorgenommen wurden. Zudem können vorzugsweise die oralen Aktoren 244 und gegebenenfalls vorhandene externe Aktoren (nicht gezeigt) von der Steuerung des externen Außengeräts aktiviert werden. Die Aktivierung erfolgt erfindungsgemäß auf Basis der Analyse und Korrelation der oralen und externen Messdaten.

Bevorzugt erfolgt bei der Auswertung und Analyse der oralen Messdaten der oralen Sensoren 216, 218, 220, 222, 228, 234 und der externen Messdaten der externen Sensoren eine Auswertung des zeitlichen Verlaufs der oralen und externen Messdaten.

Das externe Außengerät kann eine Anzeigeeinrichtung zur graphischen Darstellung der Analysen aufweisen. Das Ergebnis der Analysen kann aber auch in Form von Daten übertragen werden, beispielsweise auf ein Smartphone (nicht gezeigt) oder einen Computer (nicht gezeigt). Dort können die Ergebnisse der Analysen der Person, einem Arzt oder einem Schlaflabor zur weiteren Verwendung zur Verfügung gestellt werden. Diese können dann mittels einer Eingabeeinrichtung (nicht gezeigt) Eingriffsgrenzwerte oder bestimmte Korrelationen anpassen und beeinflussen.

Die Auswertungseinrichtung 214 des Mundstücks 201 und/oder die Auswertungseinrichtung des externen Außengeräts sind dazu eingerichtet beziehungsweise dazu programmiert, aus den ausgewerteten oralen Messdaten den Schlafzustand und Informationen zum Schlafverhalten und/oder zum Gesundheitszustand der Person abzuleiten und diese mit den externen Messdaten und/oder externen Parametern in Korrelation zu setzen und auf diese Weise Einflussfaktoren der Umgebung beziehungsweise der externen Parameter auf den Gesundheitszustand und/oder das Schlafverhalten festzustellen und zur Analyse und zur Beeinflussung des Gesundheitszustands und/oder des Schlafverhaltens mit den oralen Aktoren 244 und/oder den externen Aktoren zu nutzen. Gleichzeitig kann die Wirkung des Einsatzes der oralen Aktoren 244 und der externen Aktoren auf den Gesundheitszustand und/oder das Schlafverhalten mit Hilfe der oralen Sensoren 216, 218, 220, 222, 228, 234 gemessen werden. All diese Daten und Analysen können zur Individualisierung von Analysen späterer Schlafvorgänge der Person oder späterer Messungen gespeichert werden. Es wird im Rahmen der vorliegenden Erfindung vorgeschlagen, zur Analyse und Auswertung der umfangreichen Messdaten auf eine künstliche Intelligenz zurückzugreifen, die Muster in den Daten erkennt, und bestimmten Gesundheitszuständen und/oder Schlafzuständen zuordnet. Auf diese Weise kann ein selbstoptimierendes und selbstlernendes Gesundheitsüberwachungssystem bereitgestellt werden, das mit der Zeit immer besser auf die Gesundheitszustände und/oder die Schlafbedürfnisse der Person abgestimmt ist. Eine externe Überwachung kann auch mittels einer geeigneten Smartwatch oder einer Matte mit darin verteilten Drucksensoren zur Messung der Lage der ersten Person oder der ersten Person und der zumindest einen zweiten Person im Bett erfolgen. Ferner kann auch ein Vergleich der Messdaten der ersten Person mit den Messdaten der zumindest einen zweiten Person erfolgen, um daraus Rückschlüsse zu ziehen.

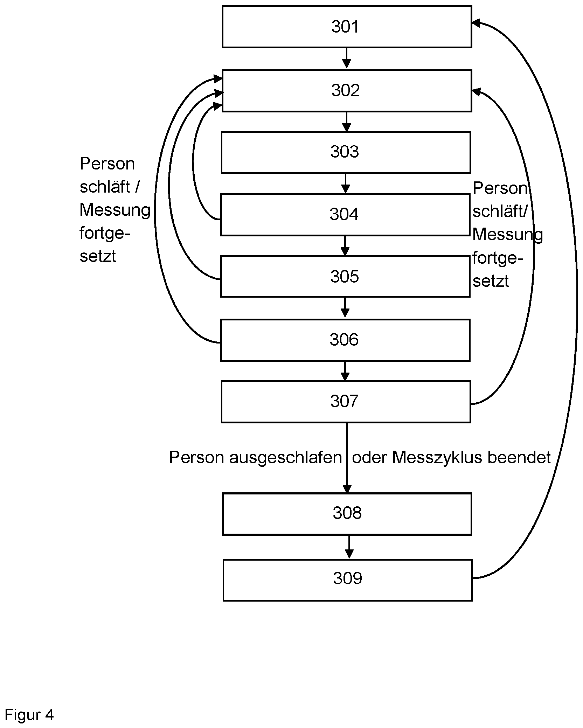

Im Folgenden wird ein beispielhaftes Verfahren anhand der Figur 1 erläutert. Der Ablauf des beispielhaften Verfahrens ist in Figur 4 schematisch dargestellt.

In einem ersten Arbeitsschritt 301 wird das Mundstück 1 in einen Mundraum einer ersten Person eingesetzt und befestigt und das Gesundheitsüberwachungssystem wird - falls notwendig - aktiviert beziehungsweise eingeschaltet. Dabei werden die oralen Sensoren 16, 18, 20, 22, 24, 26, 28, 30, 32, 34 im Mundraum der ersten Person an den gewünschten Stellen platziert. Hierzu kann das Mundstück 1 mittels der Diagnoseschiene / Aktionsschiene 10 auf dem Zahnbogen der ersten Person befestigt werden. Insbesondere können die okklusalen Seiten der Zähne des Oberkiefers und/oder des Unterkiefers der ersten Person von der Diagnoseschiene / Aktionsschiene 10 abgedeckt werden. Bevorzugt wird die Diagnoseschiene / Aktionsschiene 10 hierzu auf den Oberkiefer oder besonders bevorzugt auf die Zähne des Unterkiefers der ersten Person aufgesteckt. Gegebenenfalls werden weitere externe Sensoren 68 im Raum oder an einer zweiten Person und/oder externe Aktoren an der ersten Person angeordnet, die nicht Teil des externen Außengeräts 2 sind. Insbesondere kann das externe Überwachungsgerät 3 an einer zweiten Person, die im gleichen Raum schläft, befestigt werden, um mit den weiteren externen Sensoren 68 beziehungsweise mit einem weiteren Gesundheitsüberwachungssensor oder mittels einer externen Überwachung das Schlafverhalten der zweiten Person überwachen zu können. Die erste Person und gegebenenfalls auch die zweite Person legt sich dann üblicherweise hin, um zu schlafen. Das Verfahren kann aber auch im Wachzustand der ersten Person angewendet werden, insbesondere falls es zur Analyse des Gesundheitszustands verwendet wird. In diesem ersten Arbeitsschritt 301 kann auch ein Eichen und/oder Kalibrieren der oralen Sensoren 16, 18, 20, 22, 24, 26, 28, 30, 32, 34 des Mundstücks 1 und/oder ein Eichen und/oder Kalibrieren der externen Sensoren 56, 58, 60, 62, 64, 66, 68 durchgeführt werden.

In einem zweiten Arbeitsschritt 302 werden die zeitlichen Verläufe von oralen Parametern im Mundraum der Person mittels der oralen Sensoren 16, 18, 20, 22, 24, 26, 28, 30, 32, 34 an dem Mundstück 1 gemessen und aufgenommen. Dabei kann eine erste Auswertung der oralen Messdaten bereits im Mundstück 1 mit Hilfe der Auswertungseinrichtung 14 im Mundstück 1 erfolgen. Die zeitlichen Verläufe der oralen Parameter werden kontinuierlich über die Sender-/Empfängereinrichtung 4 des Mundstücks 1 an die Sender-/Empfängereinrichtung 6 des externen Außengeräts 2 übermittelt und im externen Außengerät 2 mit Hilfe der Auswertungseinrichtung 54 des Außengeräts 2 analysiert. Die Messung der zeitlichen Verläufe der oralen Parameter erfolgt fortlaufend auch während der nächsten Schritte.

In einem dritten Arbeitsschritt 303 werden gleichzeitig mit dem zweiten Arbeitsschritt 302 die zeitlichen Verläufe von externen Parametern im Mundraum der Person mittels der externen Sensoren 56, 58, 60, 62, 64, 66, 68 gemessen und aufgenommen. Der zeitliche Verlauf des mit dem externen Sensor 68 an der zweiten Person gemessenen externen Parameters wird kontinuierlich über die Sender-/Empfängereinrichtung 8 des externen Überwachungsgeräts 3 an die Sender-/Empfängereinrichtung 6 des externen Außengeräts 2 übermittelt und in externen Außengerät 2 mit Hilfe der Auswertungseinrichtung 54 des Außengeräts 2 analysiert. Die zeitlichen Verläufe der externen Parameter werden kontinuierlich mit Hilfe der Auswertungseinrichtung 54 des Außengeräts 2 analysiert. Die Messung der zeitlichen Verläufe der externen Parameter erfolgt fortlaufend auch während der nächsten Schritte.

In einem vierten Arbeitsschritt 304 erfolgt dann kontinuierlich und daher zeitlich überlappend mit dem zweiten Arbeitsschritt 302 und dem dritten Arbeitsschritt 303 mit Hilfe der Auswertungseinrichtung 54 des Außengeräts 2 eine Analyse und eine Korrelation der oralen und externen Parameter, um Rückschlüsse auf den Gesundheitszustand und/oder das Schlafverhalten der ersten Person sowie auf den Einfluss der externen Parameter auf den Gesundheitszustand und/oder das Schlafverhalten der ersten Person zu erlangen.

In einem fünften Arbeitsschritt 305 kann mittels der Steuerung 52 in Abhängigkeit von der im vierten Arbeitsschritt 304 erfolgten Analyse und Korrelation der zeitlichen Verläufe der oralen und externen Parameter mittels einer Aktivierung der oralen Aktoren 42, 44, 46 und gegebenenfalls von externen Aktoren der Gesundheitszustand und/oder das Schlafverhalten der ersten Person beeinflusst werden. Eine Aktivierung kann beispielsweise dann erfolgen, wenn die Zunge der ersten Person sich zu weit aus dem Mundraum in den Rachenraum bewegt, eine ungünstige Rückenlage festgestellt wird und die zweite Person sich in einer Einschlafphase befindet, obwohl und auch wenn noch kein Schnarchen im Mundraum der ersten Person gemessen wurde. Mit Hilfe der Aktoren 42, 44, 46, kann die Zunge versteift werden und einem Schnarchen vorgebeugt werden. Falls die zweite Person sich bereits im Tiefschlaf befindet, kann auf die Aktivierung der Aktoren 42, 44, 46 verzichtet werden oder erst dann erfolgen, wenn das Schnarchen eine Grenzlautstärke überschreitet und/oder mit Hilfe anderer oraler Sensoren 16, 18, 20, 22, 24, 26, 28, 30, 32, 34 beziehungsweise mit Hilfe der Analyse ein gesundheitsgefährdender Zustand erkannt wird, beispielsweise dass die Sauerstoffsättigung im Blut einen Grenzwert unterschreitet oder Atemaussetzer eine gewisse Zeitspanne überschreiten. Diese Eingriffsgrenzwerte können dabei abhängig sein von den externen Parametern und sich aus zuvor erlernten Mustern und aus Einstellungen durch die erste Person oder durch medizinisches Fachpersonal ergeben. Es ist auch möglich, im Wachzustand der Person mit den oralen Aktoren 42, 44, 46 Einfluss auf den Gesundheitszustand der Person zu nehmen, beispielsweise indem wie bei der oralen Elektroakupunktur bestimmte Nerven oder Nervenbahnen mit Hilfe elektrischer und/oder magnetischer Impulse angeregt werden und dadurch auf das Wohlbefinden und die Gesundheit der ersten Person Einfluss genommen wird. Der fünfte Arbeitsschritt 305 erfolgt vorzugsweise ebenfalls kontinuierlich und parallel zu den Arbeitsschritten zwei bis vier.

In einem sechsten Arbeitsschritt 306 erfolgen eine Überwachung und Überprüfung, ob aufgrund der Aktivierung von zumindest einem der Aktoren 42, 44, 46 sich zumindest einer der oralen Parameter ändert. Wird keine oder eine ungünstige Änderung der oralen Parameter festgestellt, so können weitere Aktoren 42, 44, 46 aktiviert werden und/oder die Aktivierung des bereits aktiven Aktors 42, 44, 46 wird verstärkt oder reduziert. Dieser sechste Arbeitsschritt 306 erfolgt zumindest zeitweise parallel zu dem fünften Arbeitsschritt 305.

In einem siebten Arbeitsschritt 307 werden alle Analysen, Reaktionen der ersten Person auf Aktivierungen der Aktoren 42, 44, 46 und Eingriffsgrenzwerte in Bezug auf das erzielte Ergebnis beziehungsweise die erzielte Wirkung hin überprüft, wobei die Auswirkungen der externen Parameter berücksichtigt werden. Basierend darauf können Eingriffsgrenzwerte verändert und angepasst werden oder Vorschläge hierzu gemacht werden, um eine Verbesserung der Einflussnahme auf den Gesundheitszustand und/oder das Schlafverhalten der ersten Person zu erzielen oder auch um den Schlaf der zweiten Person zu schonen. Auch der siebte Arbeitsschritt 307 erfolgt parallel zu den Arbeitsschritten zwei bis sechs.

In einem achten Arbeitsschritt 308 ist die erste Person erwacht oder der Messzyklus soll beendet werden. Der achte Arbeitsschritt 308 folgt nach den Arbeitsschritten zwei bis sieben. Das Mundstück 1 wird aus dem Mundraum der ersten Person entnommen und vorzugsweise gereinigt. Die externen Sensoren 56, 58, 60, 62, 64, 66, 68 können abgebaut und/oder deaktiviert werden. Der elektrische Energiespeicher 12 kann bevorzugt wiederaufgeladen werden. Der Verlauf des Schlafs der ersten Person oder der Verlauf der gesundheitsrelevanten Parameter kann auf der Anzeigeeinrichtung 70 dargestellt und/oder diese Daten an ein Smartphone oder einen Computer weitergeleitet werden. Ebenso können insbesondere alle Änderungen an den Eingriffsgrenzwerten oder Vorschläge hierzu ausgegeben werden. Auch können Hinweise auf gesundheitliche Einschränkungen und Erkrankungen ausgegeben werden, die anhand der Analysen und Reaktionen erkannt wurden.

In einem neunten Arbeitsschritt 309 können von der ersten Person und/oder von einem medizinischen Fachpersonal Eingriffsgrenzwerte oder Regelalgorithmen angepasst und verändert werden. Vor dem nächsten Schlaf oder vor dem nächsten Messzyklus wird das Verfahren erneut mit dem ersten Arbeitsschritt 301 fortgeführt. Die Wirkungen und die Auswirkungen der geänderten Eingriffsgrenzwerte und Regelalgorithmen können bei nachfolgenden Schlafvorgängen in dem sechsten Arbeitsschritt 306 und dem siebten Arbeitsschritt 307 überprüft und bewertet werden.

Der vorgenannte Ablauf des beispielhaften erfindungsgemäßen Verfahrens lässt sich mit Anpassungen auch auf das zweite erfindungsgemäße Gesundheitsüberwachungssystem nach Figur 2 und das dritte erfindungsgemäße Gesundheitsüberwachungssystem nach Figur 3 übertragen.

Die in der voranstehenden Beschreibung, sowie den Ansprüchen, Figuren und Ausführungsbeispielen offenbarten Merkmale der Erfindung können sowohl einzeln als auch in jeder beliebigen Kombination für die Verwirklichung der Erfindung in ihren verschiedenen Ausführungsformen wesentlich sein.

### Bezugszeichenliste

- 1, 101, 201: Mundstück
- 2: Externes Außengerät
- 3: Externes Überwachungsgerät
- 4, 104, 204: Sender- / Empfängereinrichtung
- 6: Sender- / Empfängereinrichtung
- 8: Sender- / Empfängereinrichtung
- 9: Befestigungsband
- 10, 110, 210: Diagnoseschiene / Aktionsschiene
- 11, 111, 211: Zahnaufnahmevertiefung
- 12, 212: Elektrischer Energiespeicher
- 14, 214: Prozessor / Auswertungseinrichtung
- 15: Steuerung
- 16, 116, 216: Oraler Sensor / Elektromyographie-Sensor
- 18, 118, 218: Oraler Sensor
- 20, 120, 220: Oraler Sensor / Oral-Neuro-Sensor
- 22, 122, 222: Oraler Sensor / Zungensensor
- 24, 124: Oraler Sensor
- 26, 126: Oraler Sensor / Bisskraftsensor
- 28, 228: Oraler Sensor / Blutdrucksensor
- 30: Oraler Sensor / Lautstärkesensor
- 32, 132: Oraler Sensor / Temperatursensor
- 34, 234: Oraler Sensor / Feuchtigkeitssensor
- 35: Ausnehmung
- 36: Hinterer lingualer Fortsatz
- 38: Vorderer lingualer Fortsatz
- 40: Proximaler Fortsatz
- 42: Aktor
- 44, 144, 244: Aktor
- 46: Aktor
- 50: Gehäuse
- 52: Prozessor / Steuerung
- 54: Prozessor / Auswertungseinrichtung
- 56: Externer Sensor / Temperatursensor
- 58: Externer Sensor / Luftdrucksensor
- 60: Externer Sensor / Luftfeuchtigkeitssensor
- 62: Externer Sensor / Mikrofon
- 64: Externer Sensor / Helligkeitssensor
- 66: Externer Sensor / Chronometer mit Kalenderfunktion
- 68: Externer Sensor / Gesundheitsüberwachungssensor für zweite Person
- 70: Anzeigeeinrichtung
- 102: Zahn
- 103: Gingiva
- 136, 236: Gaumenplatte
- 205: Lippenbändchen
- 206: Wangenbändchen
- 207: Gaumensegel
- 301: Arbeitsschritt: Einsetzen Mundstück und Aktivierung des Systems
- 302: Arbeitsschritt: Messen des zeitlichen Verlaufs der oralen Parameter
- 303: Arbeitsschritt: Messen des zeitlichen Verlaufs der externen Parameter
- 304: Arbeitsschritt: Analyse und Korrelation der oralen und externen Parameter
- 305: Arbeitsschritt: Aktivierung von Aktoren in Abhängigkeit von den Analysen und Korrelationen
- 306: Arbeitsschritt: Ermitteln von Reaktionen auf die Aktivierung der Aktoren
- 307: Arbeitsschritt: Definition neuer Zusammenhänge und Eingriffsgrenzwerte
- 308: Arbeitsschritt: Anzeige und Datenübermittlung nach dem Schlaf
- 309: Arbeitsschritt: Bewertung und Änderung von Eingriffsgrenzwerten und Regelalgorithmen

## Patentansprüche

1. Gesundheitsüberwachungssystem zur Analyse eines Gesundheitszustands einer Person, insbesondere zur Analyse eines Schlafverhaltens einer Person, oder zur Analyse und Beeinflussung eines Gesundheitszustands einer Person, insbesondere des Schlafverhaltens einer Person, das Gesundheitsüberwachungssystem aufweisend ein Mundstück (1, 101, 201) zum Einsetzen in den Mundraum der Person und eine Auswertungseinrichtung (14, 54, 214), wobei das Mundstück (1, 101, 201) zumindest einen oralen Sensor (16, 18, 20, 22, 24, 26, 28, 30, 32, 34, 116, 118, 120, 122, 124, 126, 132, 216, 218, 220, 222, 228, 234) aufweist, wobei der zumindest eine orale Sensor (16, 18, 20, 22, 24, 26, 28, 30, 32, 34, 116, 118, 120, 122, 124, 126, 132, 216, 218, 220, 222, 228, 234) zur Messung zumindest eines oralen Parameters im Mundraum der Person vorgesehen ist, wobei die Auswertungseinrichtung (14, 54, 214) zur Auswertung der oralen Messdaten des zumindest einen oralen Sensors (16, 18, 20, 22, 24, 26, 28, 30, 32, 34, 116, 118, 120, 122, 124, 126, 132, 216, 218, 220, 222, 228, 234) vorgesehen ist, wobei
das Gesundheitsüberwachungssystem zumindest einen externen Sensor (56, 58, 60, 62, 64, 66, 68) aufweist, der kein Teil des Mundstücks (1, 101, 201) ist, wobei der zumindest eine externe Sensor (56, 58, 60, 62, 64, 66, 68) zur Messung zumindest eines externen Parameters in der unmittelbaren Umgebung der Person vorgesehen ist, wobei die Auswertungseinrichtung (14, 54, 214) zur Auswertung der externen Messdaten des zumindest einen externen Sensors (56, 58, 60, 62, 64, 66, 68) vorgesehen ist, wobei die Auswertungseinrichtung (14, 54, 214) derart programmiert ist, dass eine Analyse des Gesundheitszustands und/oder des Schlafverhaltens der Person unter Berücksichtigung sowohl der Auswertung der oralen Messdaten als auch der Auswertung der externen Messdaten erfolgt.

2. Gesundheitsüberwachungssystem nach Anspruch 1, **dadurch gekennzeichnet, dass** das Gesundheitsüberwachungssystem zumindest einen Aktor (42, 44, 46, 144, 244) zur Beeinflussung des Gesundheitszustands und/oder des Schlafverhaltens der Person aufweist, wobei bevorzugt der zumindest eine Aktor (42, 44, 46, 144, 244) ein Teil des Mundstücks (1, 101, 201) ist und wobei besonders bevorzugt der zumindest eine Aktor (42, 44, 46, 144, 244) wenigstens eine Elektrode oder einen Elektromagnet zur Beeinflussung von Nerven und/oder von Muskulatur im Mundraum aufweist und/oder der zumindest eine Aktor (42, 44, 46, 144, 244) zumindest einen Motor zum mechanischen Verschieben des Unterkiefers gegen den Oberkiefer der Person aufweist.

3. Gesundheitsüberwachungssystem nach Anspruch 2, **dadurch gekennzeichnet, dass** wenigstens einer des zumindest einen Aktors (42, 44, 46, 144, 244) kein Teil des Mundstücks (1, 101, 201) ist, wobei bevorzugt der wenigstens eine des zumindest einen Aktors (42, 44, 46, 144, 244) eine Vibrationseinrichtung, eine Beleuchtung und/oder ein akustischer Signalgeber ist, und/oder
der zumindest eine Aktor (42, 44, 46, 144, 244) mit einer Steuerung (52) des Gesundheitsüberwachungssystems verbunden ist, wobei die Steuerung (52) Zugriff auf die Analyse(n) der Auswertungseinrichtung (14, 54, 214) hat und die Steuerung (52) dazu programmiert ist, den zumindest einen Aktor (42, 44, 46, 144, 244) in Abhängigkeit von der Analyse oder den Analysen der Auswertungseinrichtung (14, 54, 214) zu steuern.

4. Gesundheitsüberwachungssystem nach einem der Ansprüche 2 oder 3, **dadurch gekennzeichnet, dass**
die Person eine erste Person ist und das Gesundheitsüberwachungssystem wenigstens einen externen Schlafüberwachungssensor (68) zur Bestimmung einer Schlafphase zumindest einer zweiten Person in der Umgebung der ersten Person als einen des zumindest einen externen Sensors (56, 58, 60, 62, 64, 66, 68) aufweist, wobei die Steuerung (52) derart programmiert oder ausgestaltet ist, dass der zumindest eine Aktor (42, 44, 46, 144, 244) in Abhängigkeit von dem Schlafzyklus der zumindest einen zweiten Person gesteuert wird, wobei bevorzugt der zumindest eine Aktor (42, 44, 46, 144, 244) nicht betrieben wird, wenn sich die zumindest eine zweite Person in einer Tiefschlafphase befindet oder in einer Schlafphase befindet, in der Schnarchen oder Knirschen normalerweise als nicht störend empfunden wird, und wobei bevorzugt die Analyse des Schlafverhaltens der ersten Person und/oder eine Beeinflussung des Schlafverhaltens der ersten Person, insbesondere des Schnarchens der Person, mit dem zumindest einen Aktor (42, 44, 46, 144, 244) in Abhängigkeit von der erkannten Schlafphase der wenigstens einen zweiten Person erfolgt, und/oder
in einer Steuerung (52) des Gesundheitsüberwachungssystems zumindest ein Eingriffsgrenzwert gespeichert ist, der sich auf orale oder externe Parameter bezieht oder auf davon abgeleitete Größen, wobei die Steuerung (52) ab dem Überschreiten des zumindest einen Eingriffsgrenzwerts zumindest einen des zumindest einen Aktors (42, 44, 46, 144, 244) zur Beeinflussung des Gesundheitszustands und/oder des Schlafverhaltens der Person, insbesondere des Schnarchens der Person, aktiviert, wobei bevorzugt die der zumindest eine Eingriffsgrenzwert eine Variable ist, die von zumindest einem weiteren oralen oder externen Parameter abhängt oder von zumindest einer daraus abgeleiteten Größe, und/oder der zumindest eine Eingriffsgrenzwert in Abhängigkeit von der Analyse oder einer rechnerischen Auswertung des Gesundheitszustands und/oder des Schlafverhaltens der Person, insbesondere des Schnarchens der Person, und/oder durch manuelle Einstellung veränderbar ist.

5. Gesundheitsüberwachungssystem nach einem der vorangehenden Ansprüche, **dadurch gekennzeichnet, dass**
die Person eine erste Person ist und wenigstens ein erster externer Sensor (56, 58, 60, 62, 64, 66, 68) des zumindest einen externen Sensors (56, 58, 60, 62, 64, 66, 68) zur Messung eines Schlafzyklus wenigstens einer zweiten Person in der Umgebung der ersten Person ein Teil des Gesundheitsüberwachungssystems ist, wobei bevorzugt die Analyse des Schlafverhaltens der ersten Person und/oder eine Beeinflussung des Schlafverhaltens der ersten Person, insbesondere des Schnarchens der ersten Person, mit dem zumindest einen Aktor (42, 44, 46, 144, 244) in Abhängigkeit von dem Schlafzyklus der wenigstens einen zweiten Person erfolgt.

6. Gesundheitsüberwachungssystem nach einem der vorangehenden Ansprüche, **dadurch gekennzeichnet, dass**
der zumindest eine externe Sensor (56, 58, 60, 62, 64, 66, 68) ausgewählt ist aus der Liste: Temperatursensor (56), Luftfeuchtigkeitssensor (60), Luftdrucksensor (58), Helligkeitssensor (64), Lautstärke-Sensor, Mikrofon (62), Chronometer (66), Chronometer zur Uhrzeit-Bestimmung, Chronometer (66) zur Kalendertag-Bestimmung, Chronometer zur Mondstands-Bestimmung, Schlafzyklus-Messeinrichtung (68) zur Bestimmung des Schlafzyklus wenigstens einer zweiten Person im gleichen Raum oder in der Umgebung, Bewegungssensor, Helligkeitssensor, Sensor zur Messung der Sauerstoffkonzentration, der Staubkonzentration und/oder der CO₂-Konzentration in der Luft, Timer, wie zum Beispiel Wecker, Kamera zur Bestimmung der Position und Lage der Person, wobei bevorzugt der zumindest eine externe Sensor ein Temperatursensor, eine Uhr und/oder einen Luftdrucksensor ist, und/oder
der zumindest eine orale Sensor (16, 18, 20, 22, 24, 26, 28, 30, 32, 34, 116, 118, 120, 122, 124, 126, 132, 216, 218, 220, 222, 228, 234) ausgewählt ist aus der Liste: Lautstärkesensor (30), Bisskraftsensor (26, 126), Bewegungssensor zur Messung der Bewegung des Unterkiefers gegen die Oberkiefer, Temperatursensor (32, 132), Feuchtigkeitssensor (34, 116, 118, 120, 122, 124, 126, 132, 216, 218, 220, 222, 228, 234), Puls-Sauerstoffsensor, Vibrations- und Luftstromsensor, pH-Sensor, Lagesensor, Ultraschallsensor, Druckplattensensor, Pulslaufzeitsensor, Sensor zur Bestimmung der Färbung der Zunge und/oder der Mundschleimhäute, Atemalkoholsensor, Blutalkoholsensor, Elektromyographie-Sensor (16, 116, 216) (EMG), Elektrokardiographie-Sensor (EKG), Elektroenzephalographie-Sensor (EEG), Milchsäure-Sensor, nichtinvasiver systolischer/diastolischer Blutdrucksensor (28, 228), Blutzuckersensor, Zungensensor (22, 122, 222) zur Bestimmung der Lage der Zunge im Mundraum, Oral-Neuro-Sensor (20, 120, 220) zur Messung der neuronalen Aktivität von Zahnnerven einzelner Zähne (102) der Person, wobei bevorzugt der zumindest eine orale Sensor (16, 18, 20, 22, 24, 26, 28, 30, 32, 34, 116, 118, 120, 122, 124, 126, 132, 216, 218, 220, 222, 228, 234) einen Bisskraftsensor (26, 126), einen Zungensensor (22, 122, 222) zur Bestimmung der Lage der Zunge im Mundraum, einen Blutdrucksensor (28, 228) und/oder einen Lautstärkesensor (30) aufweist.

7. Gesundheitsüberwachungssystem nach einem der vorangehenden Ansprüche, **dadurch gekennzeichnet, dass**
die Auswertungseinrichtung (14, 54, 214) innerhalb des Mundstücks (1, 101, 201) und/oder außerhalb des Mundstücks (1, 101, 201) in einem vom Mundstück (1, 101, 201) separaten Gehäuse (50) angeordnet ist, wobei bevorzugt die Auswertungseinrichtung (14, 54, 214) zweiteilig ist und zumindest ein erster Teil (14) der Auswertungseinrichtung (14, 54, 214) zur Auswertung von den gesamten oder von Teilen der oralen Messdaten des zumindest einen oralen Sensors (16, 18, 20, 22, 24, 26, 28, 30, 32, 34, 116, 118, 120, 122, 124, 126, 132, 216, 218, 220, 222, 228, 234) innerhalb des Mundstücks (1, 101, 201) angeordnet ist und ein zweiter Teil (14, 54, 214) der Auswertungseinrichtung (54) zur Auswertung der externen Messdaten des zumindest einen externen Sensors (56, 58, 60, 62, 64, 66, 68) sowie von gegebenenfalls nicht innerhalb des Mundstücks (1, 101, 201) ausgewerteten oralen Messdaten des zumindest einen oralen Sensors (16, 18, 20, 22, 24, 26, 28, 30, 32, 34, 116, 118, 120, 122, 124, 126, 132, 216, 218, 220, 222, 228, 234) in dem separaten Gehäuse (50) angeordnet ist oder die gesamte Auswertungseinrichtung (14, 54, 214) in dem separaten Gehäuse (50) angeordnet ist, und/oder
das Mundstück (1, 101, 201) eine befüllbare Medikamentenabgabeeinrichtung als Aktor (42, 44, 46, 144, 244) aufweist, wobei die Medikamentenabgabeeinrichtung von einer Steuerung (52) des Gesundheitsüberwachungssystems in Abhängigkeit der Analyse der Auswertungseinrichtung (14, 54, 214) zur Abgabe von Medikamenten aus der Medikamentenabgabeeinrichtung steuerbar ist.

8. Gesundheitsüberwachungssystem nach einem der vorangehenden Ansprüche, **dadurch gekennzeichnet, dass**
das Mundstück (1, 101, 201) eine an die Mundraumsituation der Person individuell angepasste Diagnoseschiene (10, 110, 210), Aktionsschiene, Aufbissschiene oder Zahnspange aufweist, wobei bevorzugt die Diagnoseschiene (10, 110, 210), Aktionsschiene oder Aufbissschiene im eingesetzten Zustand flächenbündig an den okklusalen Seiten von Zähnen (102) des Oberkiefers und/oder des Unterkiefers der Person anliegt oder die Zahnspange flächenbündig an Bereichen der Zähne (102) und/oder an Bereichen der Mukosa in der Mundhöhle der Person anliegt, und/oder das Mundstück (1, 101, 201) im eingesetzten Zustand zumindest die koronalen Enden der Zähne (102) des Unterkiefers und/oder des Oberkiefers des Patienten vollständig abdeckt, bevorzugt des Unterkiefers des Patienten vollständig abdeckt, und/oder das Gesundheitsüberwachungssystem eine lernfähige künstliche Intelligenz aufweist, wobei die lernfähige künstliche Intelligenz mit der Auswertungseinrichtung (14, 54, 214) verbunden ist und zur Erkennung von Schlafmustern und/oder von Schlafstörungen in Abhängigkeit der oralen Messdaten und der externen Messdaten oder von deren Analysen eingerichtet ist, wobei bevorzugt die künstliche Intelligenz zur Steuerung zumindest eines Aktors (42, 44, 46, 144, 244) des Gesundheitsüberwachungssystems zur Beeinflussung des Gesundheitszustands und/oder des Schlafverhaltens der Person in Abhängigkeit von den erkannten Mustern, Schlafmustern und/oder von den erkannten Schlafstörungen eingerichtet ist, besonders bevorzugt in Abhängigkeit von den zuvor erlernten personenspezifischen oralen Messdaten und externen Messdaten oder von deren Analysen, bei denen bestimmte Muster, Schlafmuster und/oder Schlafstörungen erkannt wurden.

9. Gesundheitsüberwachungssystem nach einem der vorangehenden Ansprüche, **dadurch gekennzeichnet, dass**
das Mundstück (1, 101, 201) individuell an den Mundraum der Person angepasst ist, wobei bevorzugt zumindest eine äußere Form des Mundstücks (1, 101, 201) zur Verbindung mit einer Oberfläche im Mundraum der Person, insbesondere zur Verbindung mit koronalen Enden der Zähne (102) der Person, aus einem biokompatiblen Material 3D-gedruckt ist, besonders bevorzugt aus einem Polyamid 12-Material oder Poly-Laurylactam 3D-gedruckt ist, und/oder
der zumindest eine orale Sensor (16, 18, 20, 22, 24, 26, 28, 30, 32, 34, 116, 118, 120, 122, 124, 126, 132, 216, 218, 220, 222, 228, 234) zumindest bereichsweise in zumindest einen Kunststoff des Mundstücks (1, 101, 201) eingebettet ist, wobei bevorzugt ein erster Teil der Auswertungseinrichtung (14, 54, 214) zumindest in den zumindest einen Kunststoff des Mundstücks (1, 101, 201) eingebettet ist und ein zweiter Teil der Auswertungseinrichtung (14, 54, 214) in einem von dem Mundstück (1, 101, 201) separaten Gehäuse (50) des Gesundheitsüberwachungssystems angeordnet ist.

10. Gesundheitsüberwachungssystem nach einem der vorangehenden Ansprüche, **dadurch gekennzeichnet, dass**
der zeitliche Verlauf der muskulären Spannung der Zunge oder der Versteifung der Zungenmuskulatur und/oder die Atemfrequenz der Person und deren Gleichmäßigkeit mit Hilfe von wenigstens einem des zumindest einen oralen Sensors (16, 18, 20, 22, 24, 26, 28, 30, 32, 34, 116, 118, 120, 122, 124, 126, 132, 216, 218, 220, 222, 228, 234) überwachbar ist und bevorzugt von dem Gesundheitsüberwachungssystem elektronisch speicherbar und/oder von der Auswertungseinrichtung (14, 54, 214) analysierbar ist, und/oder
im Mundstück (1, 101, 201) ein elektrischer Energiespeicher (12) enthalten ist, wobei mit dem elektrischen Energiespeicher (12) der zumindest eine orale Sensor (16, 18, 20, 22, 24, 26, 28, 30, 32, 34, 116, 118, 120, 122, 124, 126, 132, 216, 218, 220, 222, 228, 234) und, falls im oder am Mundstück (1, 101, 201) vorhanden, der zumindest eine Aktor (42, 44, 46, 144, 244), eine Steuerung (52), ein Teil der Auswertungseinrichtung (14, 54, 214) und/oder eine Datenübertragungseinrichtung mit elektrischer Energie versorgbar ist, wobei bevorzugt der elektrische Energiespeicher (12) in zumindest einen Kunststoff des Mundstücks (1, 101, 201) eingebettet ist.

11. Gesundheitsüberwachungssystem nach einem der vorangehenden Ansprüche, **dadurch gekennzeichnet, dass**
das Mundstück (1, 101, 201) eine Mehrzahl von Oral-Neuro-Sensoren (20, 120, 220) zur Messung neuronaler Aktivitäten von Nerven im Mundraum als orale Sensoren (16, 18, 20, 22, 24, 26, 28, 30, 32, 34, 116, 118, 120, 122, 124, 126, 132, 216, 218, 220, 222, 228, 234) aufweist, wobei jeder Oral-Neuro-Sensor (20, 120, 220) zumindest eine Messelektrode aufweist, die mit zumindest einem der Zähne (102) oder mit einem Zahnhals oder einer Gingiva-Linie zumindest eines der Zähne (102) der Person oder der Gingiva (103) oder der Mukosa oder der Zunge der Person in unmittelbarem Kontakt stehen, wenn das Mundstück (1, 101, 201) ordnungsgemäß im Mundraum des Patienten eingesetzt ist, wobei bevorzugt die Oral-Neuro-Sensoren (20, 120, 220) zur Messung von Strömen oder elektrischen Feldern der Zahnnerven oder von Nerven in der Gingiva (103), der Mukosa oder der Zunge der Person geeignet und vorgesehen sind, wobei besonders bevorzugt jedem Zahn (102) der Person ein Oral-Neuro-Sensor (20, 120, 220) zugeordnet ist, und/oder
das Mundstück (1, 101, 201) zumindest einen Elektromagnet als den zumindest einen Aktor (42, 44, 46, 144, 244) oder als einer des zumindest einen Aktors (42, 44, 46, 144, 244) aufweist, wobei der zumindest eine Elektromagnet zum Erzeugen eines Magnetfelds im Bereich zumindest eines Nervs oder Muskels im Mundraum der Person geeignet und vorgesehen ist, wenn das Mundstück (1, 101, 201) ordnungsgemäß im Mundraum des Patienten eingesetzt ist, und/oder
das Mundstück (1, 101, 201) eine Vielzahl von Ausnehmungen (35) aufweist, insbesondere eine Vielzahl von Ausnehmungen (35) mit gleichen inneren Oberflächen aufweist, und wenigstens ein austauschbarer Sensor des zumindest einen oralen Sensors (16, 18, 20, 22, 24, 26, 28, 30, 32, 34, 116, 118, 120, 122, 124, 126, 132, 216, 218, 220, 222, 228, 234) in wenigstens einer der Ausnehmungen (35) lösbar befestigt ist, wobei bevorzugt der wenigstens eine austauschbare Sensor eine äußere Halterung aufweist, die flächenbündig mit den Ausnehmungen (35) des Mundstücks (1, 101, 201) verbindbar ist, insbesondere mit den inneren Oberflächen der Ausnehmungen (35) des Mundstücks (1, 101, 201) verbindbar ist, wenn der wenigstens eine austauschbare Sensor in der Ausnehmung (35) befestigt ist, wobei besonders bevorzugt das Gesundheitsüberwachungssystem eine Vielzahl von austauschbaren Sensoren aufweist, wobei die austauschbaren Sensoren äußere Halterungen mit gleichförmiger äußerer Oberfläche aufweisen, wobei die äußeren Oberflächen flächenbündig mit mehreren der Ausnehmungen (35) des Mundstücks (1, 101, 201) verbunden sind oder verbindbar sind, insbesondere mit den inneren Oberflächen der Ausnehmungen (35) des Mundstücks (1, 101, 201) verbunden sind oder verbindbar sind.

12. Verfahren zur Analyse eines Gesundheitszustands oder eines Schlafverhaltens einer Person oder zur Analyse und Beeinflussung des Gesundheitszustands oder des Schlafverhaltens der Person, mit den Schritten:
A) Messen eines zeitlichen Verlaufs zumindest eines oralen Parameters im Mundraum der Person und Messen eines zeitlichen Verlaufs zumindest eines externen Parameters in der Umgebung der Person;
B) rechnerische Analyse der zeitlichen Verläufe des zumindest einen oralen Parameters und des zumindest einen externen Parameters; und
C) Identifizieren von Zusammenhängen und Mustern des Gesundheitszustands oder im Schlafverhalten und/oder von Schlafzuständen der Person anhand der rechnerischen Analyse.

13. Verfahren nach Anspruch 12, **dadurch gekennzeichnet, dass** bei dem Verfahren ein Gesundheitsüberwachungssystem nach einem der Ansprüche 1 bis 11 verwendet wird.

14. Verfahren nach einem der Ansprüche 12 oder 13, **dadurch gekennzeichnet, dass** in einem Schritt D) ein Aktivieren zumindest eines Aktors (42, 44, 46, 144, 244) in Abhängigkeit von den in Schritt C) identifizierten Zusammenhängen und Mustern im Gesundheitszustand, im Schlafverhalten und/oder Schlafzuständen der Person erfolgt, wobei der zumindest Aktor (42, 44, 46, 144, 244) die Person berührt, insbesondere elektrisch, magnetisch und/oder mechanisch kontaktiert, und/oder durch elektrische und/oder magnetische Felder auf Nervenzellen der Person einwirkt, wobei bevorzugt die Wirkung eines aktivierten Aktors (42, 44, 46, 144, 244) des zumindest einen Aktors (42, 44, 46, 144, 244) auf den Gesundheitszustand, den Schlaf oder das Schlafverhalten der Person durch die fortlaufende rechnerische Analyse des zeitlichen Verlaufs des zumindest einen oralen Parameters oder durch die fortlaufende rechnerische Analyse der zeitlichen Verläufe des zumindest einen oralen Parameters und des zumindest einen externen Parameters verfolgt wird, wobei besonders bevorzugt die Intensität des zumindest einen Aktors (42, 44, 46, 144, 244) in Abhängigkeit von der nachverfolgten Wirkung des aktivierten Aktors (42, 44, 46, 144, 244) modifiziert wird, wobei insbesondere vorgesehen sein kann, dass zumindest eine Elektrode zur Stimulation wenigstens eines Muskels im Mundraum, insbesondere wenigstens eines Zungenmuskels, als Aktor (42, 44, 46, 144, 244) aktiviert wird, wenigstens ein Elektromagnet zur Beeinflussung von Nervenzellen oder Muskulatur im Bereich des Mundraums als Aktor (42, 44, 46, 144, 244) aktiviert wird und/oder dass als Aktor (42, 44, 46, 144, 244) ein Motor zum Verschieben des Unterkiefers gegen den Oberkiefer aktiviert wird.

15. Verfahren nach einem der Ansprüche 12 bis 14, **dadurch gekennzeichnet, dass** in einem Schritt E) ein personenspezifisches Erlernen von Zusammenhängen zwischen den analysierten zeitlichen Verläufen des zumindest einen oralen Parameters und des zumindest einen externen Parameters und Gesundheitszuständen und/oder Schlafzuständen der Person erfolgt, wobei bevorzugt die in Schritt E) aus vorherigen Messungen und/oder Schlafzyklen erlernten personenspezifischen Zusammenhänge in Schritt C) verwendet werden, um die Zusammenhänge und Muster im Gesundheitszustand und/oder Schlafverhalten und/oder die Schlafzustände der Person zu erkennen und/oder um die Aktivierung des Aktors (42, 44, 46, 144, 244) in Schritt D) zu steuern, und/oder aus der rechnerischen Analyse in Schritt B) zusätzlich mögliche gesundheitliche Probleme der Person abgeleitet werden und über eine Anzeigeeinrichtung (70) angezeigt oder über eine Datenübertragungseinrichtung an einen Computer oder ein Smartphone ausgegeben werden, und/oder
in Schritt C) die Analyse elektronisch gespeichert wird und/oder über eine Anzeigeeinrichtung (70) angezeigt oder dargestellt wird und/oder über eine Datenübertragungseinrichtung an ein externes Gerät an einen Computer oder ein Smartphone zum Zweck einer nachfolgenden Diagnose durch einen Arzt ausgegeben wird.

16. Mundstück (1, 101, 201) für ein Gesundheitsüberwachungssystem nach einem der Ansprüche 1 bis 11, wobei das Mundstück (1, 101, 201) zum Einsetzen in den Mundraum der Person vorgesehen ist und wobei das Mundstück (1, 101, 201) zumindest einen oralen Sensor (16, 18, 20, 22, 24, 26, 28, 30, 32, 34, 116, 118, 120, 122, 124, 126, 132, 216, 218, 220, 222, 228, 234) aufweist, wobei der zumindest eine orale Sensor (16, 18, 20, 22, 24, 26, 28, 30, 32, 34, 116, 118, 120, 122, 124, 126, 132, 216, 218, 220, 222, 228, 234) zur Messung zumindest eines oralen Parameters im Mundraum der Person vorgesehen ist, wobei eine Auswertungseinrichtung (14, 54, 214) mit dem zumindest einen oralen Sensor (16, 18, 20, 22, 24, 26, 28, 30, 32, 34, 116, 118, 120, 122, 124, 126, 132, 216, 218, 220, 222, 228, 234) verbunden oder verbindbar ist, insbesondere über eine Sendereinrichtung und/oder Sender- / Empfängereinrichtung (4, 104, 204) des Mundstücks (1, 101, 201) kabellos verbunden oder verbindbar ist.
